# EUROPEAN PATENT SPECIFICATION

(11) **EP 3 057 975 B1**
(45) Date of publication and mention of the grant of the patent: **04.09.2019**
(21) Application number: 14787034.9
(22) Date of filing: 15.10.2014
(51) Int. Cl.: C07H 11/04, C07K 9/00, C12P 19/18

(54) **N-GLYCOSYLATION OF PEPTIDES AND PROTEINS**
N-GLYCOSYLIERUNG VON PEPTIDES UND PROTEINES
N-GLYCOSYLATION DEPEPTIDES ET DE PROTEINES

(30) Priority: 16.10.2013 GB 201318313; 11.12.2013 GB 201321911
(43) Date of publication of application: 24.08.2016
(73) Proprietor: Oxford University Innovation Limited, Botley Oxford OX2 0JB (GB); Glycovaxyn AG, 8952 Schlieren (CH)
(72) Inventor: DAVIS, Benjamin G., Oxford Oxfordshire OX1 3TA (GB); VIJAYAKRISHNAN, Balakumar, Oxford Oxfordshire OX1 3TA (GB); LUI, Feng, Oxford Oxfordshire OX1 3TN (GB); WACKER, Michael, CH-8103 Unterengstringen (CH); FARIDMOAYER, Amirreza, CH-8049 Zurich (CH); KOWARIK, Michael Thomas, CH-8055 Zurich (CH)
(74) Representative: J A Kemp
(86) International application number: PCT/GB2014/053099
(87) International publication number: WO 2015/056011

(56) References cited:
- CHEN-YU LIU ET AL: "Synthesis and Evaluation of a New Fluorescent Transglycosylase Substrate: Lipid II-Based Molecule Possessing a Dansyl-C20 Polyprenyl Moiety", ORGANIC LETTERS, vol. 12, no. 7, 2 April 2010 (2010-04-02), pages 1608-1611, XP055065616, ISSN: 1523-7060, DOI: 10.1021/ol100338v
- W. HAN ET AL: "Defining Function of Lipopolysaccharide O-antigen Ligase WaaL Using Chemoenzymatically Synthesized Substrates", JOURNAL OF BIOLOGICAL CHEMISTRY, vol. 287, no. 8, 17 February 2012 (2012-02-17), pages 5357-5365, XP055158777, ISSN: 0021-9258, DOI: 10.1074/jbc.M111.308486
- ROBERT WOODWARD ET AL: "In vitro bacterial polysaccharide biosynthesis: defining the functions of Wzy and Wzz", NATURE CHEMICAL BIOLOGY, vol. 6, no. 6, 1 June 2010 (2010-06-01), pages 418-423, XP055158749, ISSN: 1552-4450, DOI: 10.1038/nchembio.351
- MARK M. CHEN ET AL: "From Peptide to Protein: Comparative Analysis of the Substrate Specificity of N-Linked Glycosylation in C. jejuni +", BIOCHEMISTRY, vol. 46, no. 18, 1 May 2007 (2007-05-01), pages 5579-5585, XP055158776, ISSN: 0006-2960, DOI: 10.1021/bi602633n
- MARK M. CHEN ET AL: "Polyisoprenol Specificity in the Campylobacter jejuni N-Linked Glycosylation Pathway +", BIOCHEMISTRY, vol. 46, no. 50, 1 December 2007 (2007-12-01), pages 14342-14348, XP055158747, ISSN: 0006-2960, DOI: 10.1021/bi701956x
- GLOVER K J ET AL: "Chemoenzymatic Synthesis of Glycopeptides with PglB, a Bacterial Oligosaccharyl Transferase from Campylobacter jejuni", CHEMISTRY AND BIOLOGY, CURRENT BIOLOGY, LONDON, GB, vol. 12, no. 12, 1 December 2005 (2005-12-01), pages 1311-1315, XP027681879, ISSN: 1074-5521 [retrieved on 2005-12-01] cited in the application

## Description

### Field of the Invention

The invention relates to a process for glycosylating proteins or peptides, and for the modification of the glycosylated proteins or peptides produced. The invention also relates to polyisoprenyl pyrophosphates for use in the process, as well as glycoconjugates obtainable via the process.

### Background to the invention

Protein glycosylation is an important co- or post-translational modification that links glycans to proteins typically through N- or O-linkages. Such modifications exist widely in eukaryotic and archaeal organisms, and greatly expand the diversity of the proteome. Whilst N-linked glycosylation had long been believed to be restricted to eukaryotic systems, the discovery of N-glycosyltransferase PglB in gram-negative bacterium *Campylobacter jejuni* highlighted greater diversity; PglB is responsible for glycosylating over 50 different proteins. Since then PglB orthologues have been found in *Campylobacter lari*, the *Helicobacter* genus (*H. pullorum, H. canadensis, H. winghamensis*) as well as *Desulfovibrio aesufuricans.*

*In vivo* PglB uses undecaprenylpyrophosphate-linked oligosaccharide as its substrate and glycosylates the primary amide nitrogen of the asparagine side chain in a D/E-X-N-X-T/S-consensus sequence, where X can be any amino acid except proline. The PglB system *in vivo* therefore is capable of N-glycosylation of proteins including this sequence via undecaprenylpyrophosphate-linked oligosaccharide.

Chen et al (Biochemistry 2007, 46, 5579-5585) describes a comparative anaylsis of the susbtrate specificity of N-Linked glycosylation in C. *jejuni.* PglB is described as central to the glycosylation pathway. In this study, a library of peptide substrates and a quantitative radioactivity-based *in vitro* assay were used to assess the amino acids of each position of the consensus glycosylation sequence for their impact on glycosylation efficiency.

Chen et al (Biochemistry 2007, 46, 14342-14348) discusses polyisoprenol specificity in the C. *jejuni* N-Linked glycosylation pathway. This study discusses the relative substrate efficiencies of polyisoprenol-modified analogues in individual steps of the glycosylation pathway.

### Summary of the Invention

The present inventors have found that the PglB system can be used to provide a flexible, biocatalytic method for *in vitro* glycoconjugate synthesis by use of a variety of chemically accessible precursors. The undecaprenylpyrophosphate precursors of the natural system are bulky and difficult to synthesise chemically. Thus previous use of the PglB system has relied on naturally occurring substrates which can be isolated from natural sources. However, such substances can generally only be obtained in nmol amounts. The present inventors have found that much shorter chain lipid pyrophosphates will also bind to PglB, thus making feasible the use of chemically synthesised substrates, rather than those isolated from natural sources. This in turn enables the use of the PglB system as a flexible and practical method for the *in vitro* glycosylation of proteins.

Accordingly, the invention provides a process for the production of a glycoconjugate by N-glycosylation of a protein or peptide comprising the sequence D/E-X-N-X-S/T, wherein each X is the same or different and is any natural amino acid other than proline,
wherein the process comprises reacting the protein or peptide with a polyisoprenyl pyrophosphate of formula (I), or a salt thereof, in the presence of PglB: 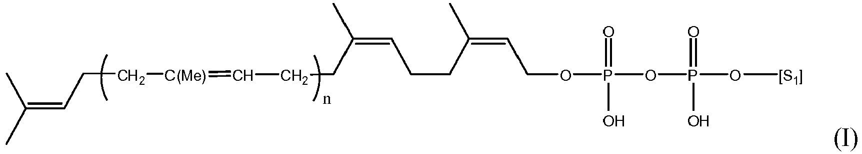
wherein n is 1, 2 or 3; and
[S1] is a monosaccharide or polysaccharide;
to produce the glycoconjugate comprising the protein or peptide having a saccharide [S1] linked to the asparagine in the sequence D/E-X-N-X-S/T.

Also provided are the lipid pyrophosphates used as substrates in this process, namely a polyisoprenyl pyrophosphate of formula (I), wherein n is 1, 2 or 3, preferably 3, and [S1] is a monosaccharide or polysaccharide wherein the saccharide unit linked to the pyrophosphate group in formula (I) is a glucose unit, and wherein the monosaccharide or polysaccharide [S1] is optionally modified at one or more positions.

The invention not only opens up the PglB system for N-glycosylation of proteins and peptides, but also provides access to a wide variety of glycoconjugates. The glycoconjugates obtained can be varied by altering the nature of the saccharide [S1], or alternatively via post-glycosylation modification of the glycoconjugates obtained by the PglB biocatalysed reaction. Thus the invention also provides a further modification step which comprises reacting the glycoconjugate with a saccharide [S2] to produce a modified glycoconjugate comprising the protein or peptide having an extended saccharide [S1-S2] linked to the asparagine in the sequence D/E-X-N-X-S/T.

Further, the inventors have found that by use of polyisoprenyl pyrophosphate substrates having saccharide units modified to include a reactive group such as an azide, alkene or alkyne, the glycoconjugate produced can be further modified to introduce additional functionality. For example, the saccharide chain [S1] can be extended by the addition of further saccharide groups.

### Brief description of the figures

Figure 1 depicts fluorescent electrophoretic analysis for N-linked peptide glycosylation reactions using different lipid carriers (Examples 1 to 4, comparative Example 5, Ref. A to C).
Figure 2 depicts MS and MSMS analysis of a glycosylated peptide.
Figure 2a provides HPLC and LC-MS results for an optimised glycosylation of Example 4 showing 92% conversion.
Figure 3 provides the results of a kinetic parameter determination of PglB with Example 1 as the lipid substrate.
Figure 4 depicts the results of a kinetic parameter determination of PglB with Example 4 as the lipid substrate.
Figure 5 depicts the results of a kinetic parameter determination of PglB with Comparative Example 5 as the lipid substrate.
Figures 6 to 8 provide LC-MS and HPLC analysis showing glycopeptide extension reactions carried out in the presence of Endo A (Examples 8 and 9, Figures 6 and 7), and β1, 4-galactotransferase (Example 10; Figure 8).
Figure 9 shows ES-MS analysis of in vitro N-linked Protein Glycosylation with Example 4.

### Detailed description of the invention

### Glycosylation process

The present invention relates to a biocatalytic process for the N-glycosylation of proteins and peptides. The process is applicable to any protein or peptide comprising the sequence D/E-X-N-X-S/T (i.e. D-X-N-X-S; E-X-N-X-S; D-X-N-X-T; or E-X-N-X-T), wherein each X is the same or different and is any natural amino acid other than proline. Thus, X may be selected from arginine, lysine, histidine, aspartic acid, glutamic acid, serine, threonine, asparagine, glutamine, cysteine, selenocysteine, glycine, alanine, valine, leucine, isoleucine, methionine, phenylalanine, tyrosine and tryptophan.

The process can be carried out with the pentapeptide D/E-X-N-X-S/T itself, or it can be carried out on a polypeptide or protein having more than five amino acid units. For example, the peptide or protein may contain up to 20 or 30 amino acids. Longer chains are also envisaged, for example up to 50, 100, 200, 300 or 400 amino acid units. The protein may be a multimeric protein in which at least one of the polypeptide chains includes the sequence D/E-X-N-X-S/T.

The process can be applied to peptides or proteins which naturally comprise the D/E-X-N-X-S/T sequence. Alternatively, the peptide or protein can be engineered to include the desired sequence. This can be achieved by standard techniques familiar to the skilled person, for example by mutagenesis. In one embodiment, the protein is an antibody.

The process is catalysed by the biocatalyst PglB. PglB is one protein in the Pgl gene cluster found in the *Campylobacter jejuni* gram-negative bacterium. PglB can be easily overexpressed in *Escherichia coli* rendering it easily available for use as an *in vitro* biocatalyst. Its expression and purification is described by Glover et al (Chem Biol 12, 1311-1315 (2005)) and Wacker et al (Science 2002, 298 (5599), 1790-1793).

The glycosylation reaction is typically carried out under ambient conditions, for example at a pH of about 7 to about 8 and at room temperature. The peptide and polyisoprenyl pyrophosphate substrate react in a ratio of 1:1. Accordingly, the peptide and polyisoprenyl pyrophosphate are therefore typically used in approximately a 1:1 ratio. Providing an increased amount of the polyisoprenyl pyrophosphate substrate has however been found to increase the yield of glycosylated peptide. Accordingly, the preferred ratio of peptide: polyisoprenyl pyrophosphate substrate is 1:>1, e.g. 1:at least 2, 1:at least 3, or about 1:10. The substrate in the reaction described herein is a compound which is a polyisoprenyl pyrophosphate of formula (I) or a salt thereof. The compound contains multiple isoprenyl units. When n is 1, there are 4 isoprenyl units. Preferably n is 1, 2 or 3, most preferably n is 3.

The two isoprenyl units linked to the phosphate groups are in *cis* form. Previous work has focused on longer isoprenyl chains. However, the present inventors have determined that as long as the two isoprenyl units linked to the phosphate group are in cis form, much shorter isoprenyl chains can be used. This is of significant benefit as the shorter chains are accessible by synethic routes with a small number of steps.

Isoprenyl units more distant from the phosphate groups (i.e. the third or further unit from the phosphate groups) may be in either cis or trans form, or a mixture of cis and trans form units may be present. For example, where Z is a cis-isoprenyl unit and E is a trans isoprenyl unit, with ω being the terminal isoprenyl unit, the isoprenyl chain may be of formula:
ωZZZ-, ωEZZ-, ωZZZZ-, ωEZZZ-, ωZEZZ-, ωEEZZ-, ωEZZZZ-, ωZZZZZ-, ωEEZZZ-, ωZEZZZ-, ωZEEZZ-, ωEEEZZ-, ωEZEZZ- and ωZZEZZ-. Particular examples include ωZZZ-, ωEZZ, ωEZZZZ- and ωZZZZZ-.

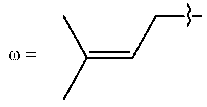

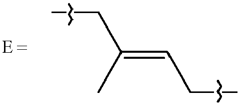

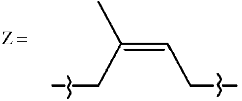

The isoprenyl chain is linked to the saccharide of the substrate via a pyrophosphate group. The pyrophosphate may be in free acid form or in the form of a salt. Typically, a salt is used. Salts may be formed with any appropriate base including inorganic and organic bases. Examples of suitable bases include alkali metal hydroxides e.g. sodium hydroxide, alkaline earth metal hydroxides e.g. magnesium hydroxides, ammonia, amines including trialkylamines, e.g. triethylamine and cyclic nitrogen containing bases e.g. imidazole. Particular examples are ammonia, amines including trialkylamines, e.g. triethylamine and cyclic nitrogen containing bases e.g. pyridine, imidazole, triazole and tetrazole, most especially ammonia and trialkylamines, e.g. triethylamine. Thus, for example, salts may be formed with cations such as ammonium and tetrabutylammonium.

The substrate comprises a saccharide group [S1] which will be transferred to the Asn side chain of the peptide or protein in the N-glycosylation reaction. As used herein a saccharide, saccharide chain or saccharide group is made up of one or a plurality of saccharide units. A saccharide unit as used herein is a monosaccharide, typically a hexose or pentose, preferably a hexose. D-form saccharide units are preferred. A polysaccharide is a saccharide containing two or more saccharide units. The term "polysaccharide" as used herein therefore includes oligosaccharides, which typically have a small number of saccharide units, e.g. from 2 to 9 saccharide units, typically from 3 to 9 saccharide units. The nature of the saccharide group for use in the substrates described herein can vary widely, although typically monosaccharides or disaccharides are employed. Monosaccharides are used in one aspect as the synthesis of the substrate is simplified. However, saccharide chains of more than 2 saccharide units may be used, for example 3, 4, 5, 6 or 7 saccharide units may be present. In one aspect of the invention, therefore [S1] is an oligosaccharide.

The nature of the saccharide units present in [S1] can vary and where [S1] is a polysaccharide, the saccharide units may be the same or different. For example, glucose, mannose, galactose, fucose, rhamnose, heptose and/or 2-keto-3-deoxyoctonate (KDO) units may be used. Glucose, fucose, rhamnose, heptose and/or 2-keto-3-deoxyoctonate (KDO) units are preferred, in particular glucose. Typically, the saccharide unit linked to the pyrophosphate group (and which will be linked to the protein or peptide following glycosylation) is a glucose unit. Further, the second saccharide unit (i.e. the saccharide unit second in the chain when moving away from the pyrophosphate group) is typically a glucose unit. Thus, the saccharide unit [S1] may comprise Glc or Glc-Glc as the first two saccharide units, for example [S1] may be Glc or Glc-Glc.

Each saccharide unit may optionally be modified at one of more positions in a manner which would be familiar to the skilled chemist. Modifications to the saccharide, when present, are typically at one or more of positions 2, 3, 4 or 6. A common modification is the replacement of an OH group on the saccharide unit with NHAc. Alternative modifications are also envisaged. For example, an OH group on the saccharide can be replaced with H, F, Cl, Br, I, an alkyl chain, e.g. Me or Et, or an alkoxy group, e.g. OMe or OEt. Alternatively or additionally, a H atom on the saccharide can be replaced with an OH, F, Cl, Br, I, an alkyl chain, e.g. Me or Et, or an alkoxy group, e.g. OMe or OEt. Any one or combination of two or more such modifications may be made.

In one aspect of the invention, the saccharide unit linked to the pyrophosphate group (and which will be linked to the protein or peptide following glycosylation) is a glucose unit modified by replacement of OH with NHAc (GlcNAc). Typically the C2 carbon atom is modified. Further, the second saccharide unit, where present, is typically a glucose unit modified to carry NHAc (GlcNAc). Typically the C2 carbon atom is modified. Thus, the saccharide unit [S1] may comprise GlcNAc or GlcNAc-GlcNAc as the first two saccharide units, for example [S1] may be GlcNAc or GlcNAc-GlcNAc. Further modifications may optionally be present in addition to the NHAc group.

### Use of saccharides bearing reactive groups

The saccharide [S1] may be modified to carry one or more groups R1, wherein each R1 comprises a reactive group. The reactive group(s) can be used to introduce further functionality in the glycoconjugate via post-glycosylation reaction of the glycoconjugate. for example, the saccharide group [S1] can be extended to provide a longer saccharide chain by reaction of one or more groups R1 with a further saccharide which bears a group R2 capable of reacting with R1. In an alternative, preferred embodiment which is described in more detail below, post-glycosylation reaction can be achieved by reaction of one or more OH groups on the saccharide with a group R2 capable of reacting with OH. In this case, post-glycosylation reaction can take place without further modification of [S1] to carry a reactive group.

The invention therefore provides a process for the production of a glycoconjugate by N-glycosylation of a protein or peptide comprising the sequence D/E-X-N-X-S/T, wherein each X is the same or different and is any natural amino acid other than proline,
wherein the process comprises reacting the protein or peptide with a polyisoprenyl pyrophosphate of formula (I), or a salt thereof, in the presence of PglB: 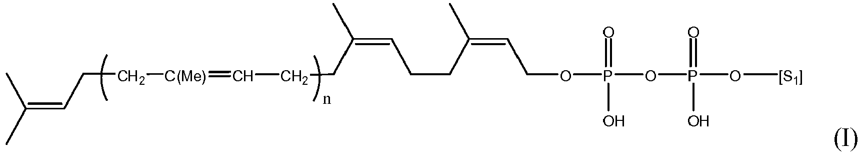
wherein n is 1, 2 or 3; and
[S1] is a monosaccharide or polysaccharide which is modified to carry one or more groups R1, wherein R1 comprises a reactive group;
to produce the glycoconjugate comprising the protein or peptide having a saccharide [S1] linked to the asparagine in the sequence D/E-X-N-X-S/T.

In this aspect of the invention, the lipid chain is a short polyisoprenyl chain wherein n is 1, 2 or 3, most preferably 3.

Typically, [S1] is modified to carry one or two groups R1, for example one group R1. R1 comprises a reactive group and optionally a linker moiety L. Thus, in one aspect the reactive group is bonded directly to the saccharide. Alternatively, the reactive group is bonded to the saccharide via a linker moiety L. Where a linker moiety is present, R1 may comprise two or more reactive groups. Typically, one or two, for example one, reactive group is present on a single R1 group. R1 may be present in addition to one or more further modifications on the saccharide. For example, the saccharide may comprise GlcNAc or GlcNAc-GlcNAc, wherein one of the GlcNAc groups (for example the terminal GlcNAc group) is further modified to carry a group R1.

Suitable reactive groups are well known to those skilled in the art of sugar chemistry and include typical reactive groups used in so called "click" chemistry. Examples of reactive groups R1 include azide groups (-N₃) which can react with an alkyne group (HC≡C-) at R2; alkyne groups (HC≡C) which can react with an azide (N₃) at R2; and alkenes ( H₂C=C-) which can be used in radical type reactions with other alkenes at R2.

The linker moiety L, when used, may be any appropriate non-reactive linker which serves to move the reactive group further from the saccharide. Suitable examples include alkylene moieties, -oxy-alkylene- moieties and moieties of formula -NH-C(=O)-alkylene-. Further examples of possible linker moieties include -NH-C(=O)-O-alkylene-, -O-C(=O)-alkylene and -O-C(=O)-O-alkylene-. Typically, the alkylene moiety is a C₁-C₆ alkylene moiety, for example a C₁-C₄ alkylene moiety, for example methylene or ethylene, in particular methylene. Typically the alkylene group is unsubstituted. Thus, R1 may be of formula Y, -(C₁-C₆ alkylene)-Y, -O-(C₁-C₆ alkylene)-Y, -NH-C(=O)-(C₁-C₆ alkylene)-Y, -NH-C(=O)-O-(C₁-C₆ alkylene)-Y, -O-C(=O)-(C₁-C₆ alkylene)-Y or -O-C(=O)-O-(C₁-C₆alkylene)-Y, in particular Y, -(C₁-C₆ alkylene)-Y, -O-(C₁-C₆ alkylene)-Y or -NH-C(=O)-(C₁-C₆ alkylene)-Y, wherein Y is a reactive group, e.g. -N₃, -CH=CH₂ or HC≡C-.

Where more than one reactive group is present on any R1, the reactive groups may be the same or different. Further, when more than one R1 group is present, the groups R1 may be the same or different. The presence of two reactive groups enables the skilled person to introduce functionality at two separate positions on the saccharide. If different reactive groups are used, this has the benefit that they can be employed in separate synthetic steps without the need for protective groups to be used.

### Post-glycosylation reactions

A glycoconjugate comprising a saccharide [S1] which optionally carries one or more reactive groups can be used in a further reaction to introduce functionality to the saccharide, or to extend the saccharide chain. In the case of the extension of the saccharide chain, an example of a synthetic scheme for this reaction is as follows:

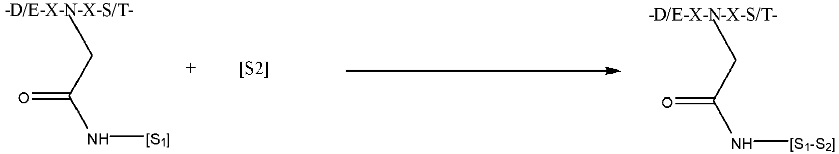

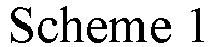

In scheme 1, [S1] is a mono- or polysaccharide wherein the saccharide group is optionally modified to carry one or more group(s) R1. The saccharide [S1] may contain other modifications, for example the saccharide may be GlcNAc, or it may be GlcNAc which is modified at C3, 4 or 6 (e.g. C6) to carry a reactive group. [S2] is a mono- or polysaccharide capable of reacting with [S1]. Typically, [S2] is modified to carry one or more group(s) R2, typically one group R2. R2 comprises at least one reactive group which is capable of reacting with R1 or with OH. Thus for example as described above when R1 comprises an azide, R2 typically comprises an alkyne group. The reaction of [S1] with [S2] therefore provides an extended saccharide chain [S1-S2] having a covalent link between the two saccharides formed by the reaction between R1 and R2, or by the reaction between OH and R2.

[S2] typically comprises one or more, for example at least two or at least 3 saccharide units. [S2] may have up to 10 saccharide units, for example up to 8 saccharide units, for example 5 saccharide units. The nature of the saccharide units present in [S2] can vary and where [S2] is a polysaccharide, the saccharide units may be the same or different. For example, glucose, mannose, galactose, fucose, rhamnose, heptose, sialic acid and/or 2-keto-3-deoxyoctonate (KDO) units may be used. An example of a suitable [S2] is the tetrasaccharide Man-Man-Man-GlcNAc, wherein the GlcNAc is modified to carry a group R2.

[S2] is typically modified such that it carries at least one group R2. One or more, typically one, group R2 may be present. Typically, R2 is carried on a saccharide unit which terminates the saccharide chain. The reactive group R2 comprises a reactive group which is capable of reacting with R1 or with OH. Thus, examples of reactive groups on R2 include azide groups (-N₃) which can react with an alkyne group (HC≡C) at R1; alkyne groups (HC≡C) which can react with an azide (N₃) at R1; and alkenes ( H₂C=C-) which can be used in radical type reactions with other alkenes at R1.

In the case where R2 comprises a reactive group capable of reacting with OH, the reaction may be carried out by use of, for example, endoglycosidase or glycosyltransferase catalysed reaction. Such reactions are described, for example, by Lomino et al (Bioorganic & Medicinal Chemistry 21 (2013) 2262-2270) and Schwarz et al (Nature Chemical Biology 6 (2010) 264-266) For instance, suitable reactive groups on R2 include oxazoline, which can be reacted with an OH on the saccharide via an endoglycosidase (e.g. ENDO-A, ENDO-S or ENDO-M) catalysed reaction. An oxazoline ring is typically linked to the saccharide [S2] via two adjacent positions in the saccharide ring, thus forming a fused ring system.

Such trans-glycosylation reactions between an OH on [S1] and a further saccharide [S2] can be carried out by any means known to the skilled person. A further example of a suitable reaction is the use of glycosyltransferase-catalysed reactions. For example, uridine diphosphate galactose (UDP-galactose) may be used as the saccharide [S2] and reaction with [S1] may be via galactose transferase (GalT, e.g. β-1,4-GalT) catalysed reaction. In this example, the UDP group forms the reactive group R2 on saccharide [S2].

R2 comprises a reactive group and optionally a linker moiety. Thus, in one aspect the reactive group is bonded directly to the saccharide, a reactive group which is a ring being optionally fused to the saccharide. Alternatively, the reactive group is bonded to the saccharide via a linker moiety. Where a linker moiety is present, R2 may comprise two or more reactive groups. Typically, one or two, for example one, reactive group is present on a single R2 group.

The linker moiety, when used, may be any appropriate non-reactive linker which serves to move the reactive group further from the saccharide. Suitable examples include alkylene moieties, -oxy-alkylene moieties and moieties of formula -NH-C(=O)-alkylene-. Further examples of possible linker moieties include -NH-C(=O)-O-alkylene-, -O-C(=O)-alkylene and -O-C(=O)-O-alkylene-. Typically, the alkylene moiety is a C₁-C₆ alkylene moiety, for example a C₁-C₄ alkylene moiety, for example methylene or ethylene, in particular methylene. Typically, the alkylene moiety is unsubstituted. Thus, R2 may be of formula Y, -(C₁-C₆ alkylene)-Y, -O-(C₁-C₆ alkylene)-Y, -NH-C(=O)-(C₁-C₆ alkylene)-Y, -NH-C(=O)-O-(C₁-C₆ alkylene)-Y, -O-C(=O)-(C₁-C₆ alkylene)-Y or -O-C(=O)-O-(C₁-C₆alkylene)-Y, in particular Y, -(C₁-C₆ alkylene)-Y, -O-(C₁-C₆ alkylene)-Y or -NH-C(=O)-(C₁-C₆ alkylene)-Y, wherein Y is a reactive group, e.g. -N₃, -CH=CH₂ or HC≡C-.

Where more than one reactive group is present on any R2, the reactive groups may be the same or different. Further, when more than one R2 group is present, the groups R2 may be the same or different. Typically, only one group R2 is present. Further R2 typically comprises a single reactive group.

In addition to the group(s) R2, each saccharide unit in [S2] may optionally be further modified at one or more positions in a manner which would be familiar to the skilled chemist. A common modification is the replacement of an OH group on the saccharide unit with NHAc. Alternative modifications are also envisaged. For example, an OH group on the saccharide can be replaced with H, F, Cl, Br, I, an alkyl chain, e.g. Me or Et, or an alkoxy group, e.g. OMe or OEt. Alternatively or additionally, a H atom on the saccharide can be replaced with an OH, F, Cl, Br, I, an alkyl chain, e.g. Me or Et, or an alkoxy group, e.g. OMe or OEt. Alternative modifications may also be used to introduce further functionality into the saccharide. Any one or combination of two or more such modifications may be made. Modifications may be at any available position on the saccharide unit.

An example of modifications to introduce functionality into [S2] include modifications to include labelling groups, for example radioactive labels. For example, [S2] may be modified to include 18-F, 64-C or 131-1. Alternative labelling groups may be incorporated into the saccharide if desired, for example fluorophores.

The process for carrying out the post-glycosylation reaction will vary dependent on the reactive groups involved. In the case of the attachment of a group [S2], the reaction can be carried out in accordance with known methods for transglycosylation.

### Synthesis of substrates

The substrates for the glycosylation reaction can be produced in accordance with Scheme 2 below:

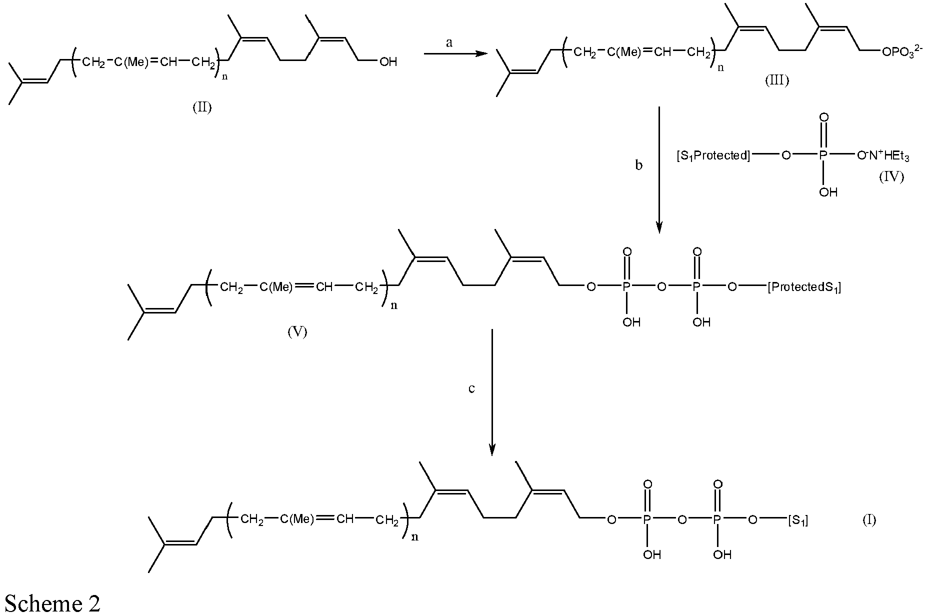

In step a, the polyprenol compound (II) is phosphorylated in the presence of, for example, mono(tetrabutylammonium) phosphate (TBAP) activated with trichloroacetonitrile (TCA) or other suitable phosphorylating agent. In step b, the polyisoprenylphosphate (III) is linked to protected saccharide-phosphate compound (IV) using carbonyldiimidazole (CDI)-mediated phosphoesterification. Any suitable protecting groups may be used, for example OH groups may be protected as OAc groups. In step c the saccharide of compound (V) is deprotected to yield the substrate (I), for example by deacetylation with catalytic NaOMe in MeOH (Zemplen conditions). Specific examples of substrates produced in accordance with this scheme are given in the examples section below.

The polyisoprenol starting material may be built up from commercially available materials using a series of building blocks. This is exemplified in Scheme 3:

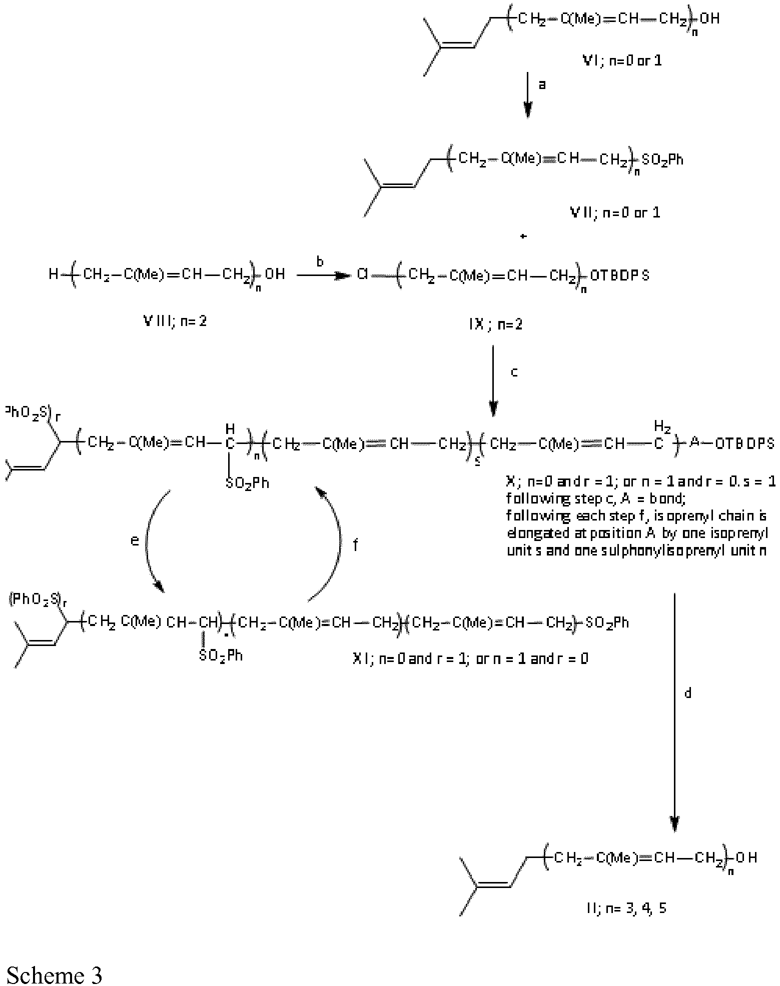

Two basic building blocks are used, created from isoprenol or short polyisoprenols. Firstly, alcohol VI is converted to sulfone VII in step a by reaction with PBr₃ followed by PhSO₂Na to create a first "head" building block. The "tail" building block IX is generated in step b from the corresponding alcohol VIII by reaction with TBDPSC1 and imidazole followed by (i) SeO₂; (ii) NaBH₄; and (iii) methanesulfonyl cholride. The "head" and "tail" are then reacted together in step c in the presence of BuLi/1,3-dimethyl-3,4,5,6-tetrahydro-2(1H)-pyrimidinone (DMTP) to generate the extended polyprenyl compound X.

If need be, X can be elongated by the addition of further building blocks. For example, X can be converted to a sulfone in step e by reaction with tetrabutylammonium fluoride followed by (i) methanesulfonyl chloride and (ii) NaSO₂Ph; and subsequently reacted in step f with a further "tail" building block IX to generate a further extended polyprenyl system X. Elongation steps e and f can be repeated if need be to generate the required length of polyprenyl chain. Each repetition of steps e and f introduces two additional isoprenyl units into the chain at position A, one of the isoprenyl units bearing the phosphoryl group, SO₂Ph.

Once the desired number of isoprenyl units is reached, X can be converted to the desired polyisoprenol (II), by a single step removal of sulfonyl groups using LiEt₃BH/(dppp)PdCl₂.

The saccharide starting material can be produced by phosphorylation of an appropriate saccharide [S1]. Specific examples of such phosphorylation are provided in the examples section herein. Saccharides [S1] are commercially available or can be produced by techniques known to the skilled chemist.

### Glycoconjugates and modified glycoconjugates

Also described are glycoconjugates obtainable by the methods described herein, wherein the glycoconjugates are produced from substrates having a saccharide [S1] which carries one or more reactive groups. Modified glycoconjugates are also described which are obtainable by the process for producing a modified glycoconjgate as defined herein.

For instance, the glycoconjugate may be a glycoconjugate or modified glycoconjugate comprising a peptide or protein comprising the sequence D/E-X-N-X-S/T, wherein each X is the same or different and is any natural amino acid other than proline, wherein the peptide or protein is N-glycosylated on the asparagine amide group to carry a saccharide [S]: 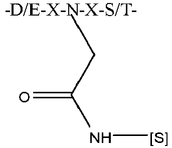 wherein [S] is a monosaccharide or polysaccharide [S1] as defined herein which carries at least one reactive group, or an extended saccharide [S1-S2] obtainable by reacting a saccharide [S1] with a further saccharide [S2].

The protein or peptide, and saccharides [S1] and [S2] are preferably as described further herein with reference to the described process.

The invention will be further described below with reference to a number of examples, but is not intended to be limited to these examples:

### Examples

Proton nuclear magnetic resonance (¹H NMR) spectra were recorded on a Bruker AVII500 (500 MHz) or a Varain Mercury VX 400 (400 MHz) spectrometer, as indicated. Carbon nuclear magnetic resonance (¹³C NMR) spectra were recorded on a Bruker AVII500 (125.8 MHz) or a Varain Mercury VX 400 (100.6 MHz) spectrometer, as indicated. Phosporous nuclear magetic resonance (³¹P NMR) spectra were recorded on a Bruker AVII500 (202 MHz) or a Varain Mercury VX 400 (162 MHz) spectrometer, as indicated. NMR spectra were fully assigned using COSY, HSQC, HMBC. All chemical shifs are quoted on δ scale in ppm using residual solvent as the internal standard. Coupling constants (*J*) are reported in Hz with the following splitting abbreviations: s = singlet, d = doublet, t = triplet, q = quarter, app = apparent.

Low resolution mass spectra (LRMS) were revorded on a Waters Micromass LCT premier TOF spectrometer using electrospray ionization (ESI-MS) and high resolution mass spectra (HRMS) were recorded on a Bruker MicroTOF ESI mass spectrometer.

Melting points (m.p.) were recorded on a Leica Galen III hot stage microscope equipped with a Testo 720 thermocouple probe and are uncorrected. Optical rotation were measured on a Perkin-Elmer 241 polarimeter with a path length of 1.0 dm and are reported with implied units of 10⁻¹ deg cm² g⁻¹. Concentrations (c) are given in g/100 ml. Thin layer chromatography (TLC) was carried out using Merck aluminium backed sheets coated with 60F254 silica gel. Visualization of the silica gel plates was achieved using a UV lamp (λₘₐₓ = 254 nm) and/or dipping the plate in ceric ammonium molybdate (CAM) stain follwed by heating.

### Reference Example 1: (Z)-1-Benzenesulfonyl-3,7-dimethylocta-2,6-diene (9)

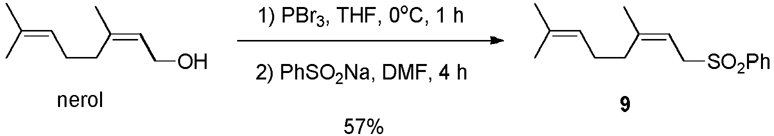

To a solution of nerol (1 mL, 5.7 mmol) in THF (2 mL) was added PBr₃ (268 µL, 2.86 mmol) at 0°C. The solution was stirred at 0°C for 15 min, and was then diluted with ether/water and washed with NaHCO₃, water, brine and dried over Na₂SO₄. Solvent was removed to give a brown oil which was used in the next step without purification.

Neryl bromide was added to a solution of NaSO₂Ph (1.4 g, 8.5 mmol) in DMF (20 mL) at 0°C. The reaction mixture was stirred at the same temperature for 4 h and was then diluted with EtOAc, washed with 1 M HCl, NaHCO₃, water, brine and dried over Na₂SO₄. Solvent was removed *in vacuo* to give a yellow oil, which was then purified by silica gel column chromatography (EtOAc: petroleum ether = 15:85, R_{f}=0.2) to give (*Z*)-1-benzenesulfonyl-3,7-dimethylocta-2,6-diene (**9**) as a colourless oil.

### Reference Example 2: (E)-1-Benzenesulfonyl-3,7-dimethylocta-2,6-diene (10)

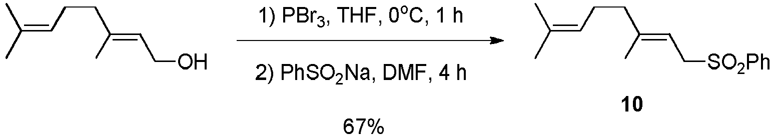

Geraniol (6.16 g, 0.04 mol) was dissolved in 10 mL of THF. PBr₃ (1.9 mL, 0.02 mol) was added dropwise at 0°C. The solution was stirred at 0°C for 1 h, and was then diluted with ether and washed with NaHCO₃, water, brine and dried over Na₂SO₄. Solvent was removed to give geranyl bromide as a brown oil (7.7 g, 90%) which was used in the next step without purification.

Geranyl bromide (7.7g, 0.035 mol) was added to a solution of NaSO₂Ph (8.74 g, 0.053 mol) in DMF (20 mL) at 0°C. The reaction mixture was stirred at the same temperature for 4 h and was then diluted with EtOAc, washed with 1 M HCl, NaHCO₃, water, brine and dried over Na₂SO₄. Solvent was removed *in vacuo* to give a yellow oil, which was then purified by silica gel column chromatography (EtOAc: petroleum ether = 15:85, R_{f}=0.2) to give (*E*)-1-benzenesulfonyl-3,7-dimethylocta-2,6-diene (**10**) as a colourless oil (7.4g, 75%).

### Reference Example 3: (2Z, 6Z)-1-(tert-Butyldiphenylsilyloxy)-8-chloro-3,7-dimethylocta-2,6-diene (12)

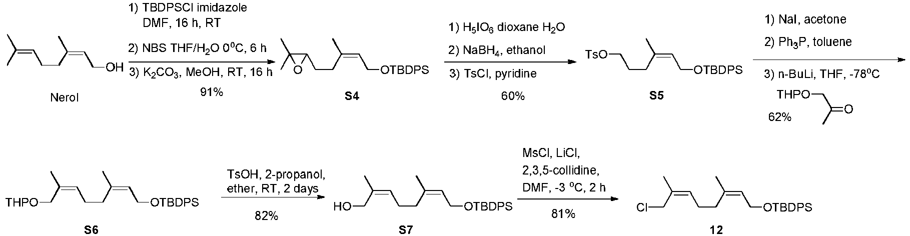

To a DMF solution (15 mL) containing imidazole (3.74 g, 0.055 mol) and nerol (8.75 mL, 0.05 mol) was added TBDPSCl (13.6 mL, 0.0525 mL). The reaction was stirred at RT for 16 h. Reaction mixture was diluted with 200 mL of EtOAc and washed with 0.5 M HCl, saturated NaHCO₃, water, brine and dried over Na₂SO₄. Crude neryl TBDPS ether was yielded as colourless oil (19.2 g, 98%).

To a solution of the crude neryl TBDPS ether (18.7 g, 0.048 mol) in THF/H₂O (100/33 mL) at 0 °C was added *N*-bromosuccinimide (9.35 g, 0.053 mol). The reaction mixture was stirred at 0 °C for 6 h. TLC showed that >95% of the starting material had been consumed (EtOAc : petroleum ether = 5:95, R_{f}=0.07). THF was removed *in vacuo* and the organic layer was extracted with diethyl ether, which was then washed with H₂O, saturated NaHCO₃, brine and dried over Na₂SO₄. Solvent was removed to give a yellow oil, which was used in the next step without purification.

The yellow oil was re-dissolved in methanol (100 mL) and K₂CO₃ (12.7 g, 0.092 mol) was added. The reaction mixture was stirred at RT for 16 h, and TLC showed that >95% of the starting material had been converted to the product (EtOAc : petroleum ether = 15:85, R_{f}=0.39). The reaction mixture was then filtered and methanol was removed *in vacuo.* The resultant oil was re-dissolved in petroleum ether and was filtered and concentrated to give (*Z*)-1-(*tert*-butyldiphenylsilyloxy)-6,7-epoxy-3,7-dimethylocta-2-ene (**S4**) as a colourless oil (18.03 g, 93%), which was used in the next step without further purification.

Epoxide **S4** (18.03 g, 0.044 mol) was dissolved in dioxane/ H₂O (20 mL/ 10 mL) and was added H₅IO₆ (12.5g 0.055 mol). The reaction was stirred at 0 °C for 12 h before it was filtered through celite. The reaction mixture was then extracted with EtOAc (3 x 50 mL). The EtOAc layer was extracted with NaHCO₃, brine and dried over NaSO₄ and solvent removed. The resulting yellow oil was purified by silica gel column chromatography (EtOAc : petroleum ether = 15:85, R_{f}=0.33) to give the aldehyde as colourless oil (13.4 g, 73% from **S4**).

The aldehyde (11 g, 0.03 mol) was dissolved in EtOH (50 mL) and NaBH4 (2.16 g, 0.057 mol) was added. The reaction was stirred at 0°C for 3 h and was carefully quenched by addition of 0.5 M HCl. The reaction mixture was filtered through celite, concentrated and re-dissolved in diethyl ether. The ether solution was washed with brine, dried over Na₂SO₄ and concentrated. Purification using silica gel column chromatography (EtOAc : petroleum ether = 15:85, R_{f}=0.1) gave (*Z*)-1-*O*-*tert*-butyldiphenylsilyl-3-methylhexa-2-en-1,6-diol as colourless oil (10.2 g, 94%).

The hydroxyl compound (7.3 g, 0.02 mol) was dissolved in pyridine (15 mL), to which TsCl (4.6 g, 0.024 mmol) was added. The reaction was carried out at room temperature under argon for 3 h and was shown to be complete by TLC (EtOAc : petroleum ether = 15:85, R_{f}=0.29). The reaction mixture was concentrated *in vacuo* and re-dissolved in petroleum ether. After filtration of the insoluble pyridinium salt, the organic layer was successively washed with CuSO₄ solution, water, saturated NaHCO₃, brine and was dried over Na₂SO₄. Solvent was removed, and purification using silica gel column chromatography (EtOAc : petroleum ether = 3:7, R_{f}=0.5) gave (*Z*)*-*1*-O-tert-*butyldiphenylsilyl-3-methyl-6-*O*-(4-toluenesulfonyl)hexa-2-en-1,6-diol (**S5**) as colourless oil (9. 1 g, 87%).

**S5** (10.2 g, 20 mmol) and NaI (7.5 g, 50 mmol) was refluxed in 30 mL of dry acetone for 16 h. The reaction was shown to be completed by TLC (EtOAc : petroleum ether = 15:85, R_{f}=0.43). The reaction mixture was poured into Na₂S₂O₃, and was extracted with petroleum ether, which was dried over Na₂SO₄ and evaporated to give the iodinated compound as a colourless oil (9.4 g, crude, 98%) and was used in the next step without purification.

The iodinated compound was dissolved in dry toluene (40 mL), and was added PPh₃ (7.2 g, 27 mmol). After reflux under argon for 20 h, the reaction mixture was diluted in petroleum ether (150 mL) and filtered. The insoluble were collected and dissolved in acetone and rotovapped to a volume of 5 mL. 100 mL of boiling petroleum ether was added and the mixture was allowed to crystallise at 4 °C. The phosphonium salt was obtained as off white crystal after filtration (8.35 g, 70 %).

The phosphonium salt (8.3 g, 11.2 mol) was dissolved in 40 mL of THF and was added 1.45 M BuLi (9 mL, 13 mmol). After stirring for 1 h, the mixture was cooled to -78 °C, and THP-acetol (2.4 g, 15 mmol) in 10 mL THF was added. The mixture was allowed to warm up to RT over 16 h and was then poured into water. The reaction mixture as extracted with petroleum ether, dried with anhydrous Na₂SO₄ and concentrated. Silica gel column chromatography (EtOAc: petroleum ether = 15:85, R_{f}= 0.53) gave (2*Z*, 6*Z*)-1-(*tert-*butyldiphenylsilyloxy)-3,7-dimethyl-8-(2-tetrahydropyranyl oxy)-octa-2,6-diene (**S6**) as colourless oil (5.1 g, 91%).

**S6** (4 g, 8.12 mmol) was dissolved in 2-propanol/Et₂O (20 mL/10 mL), to which was added *p*-toluene sulfonic acid (35 mg), the reaction was stirred at RT for 3 days, and was then diluted with Et₂O (250 mL), extracted with saturated NaHCO₃ solution, brine and dried over anhydrous Na₂SO₄. Evaporation followed by silica gel column chromatography (EtOAc: petroleum ether = 15:85, R_{f}= 0.2) gave (2Z, 6*Z*)-1-*O*-(*tert*-butyldiphenylsilyl)-3,7-dimethylocta-2,6-dien-1,8-diol (**S7**) as colourless syrup (3.27 g, 82%) as well as the starting material **S6** (0.59 g, 15%).

Methanesulfonyl chloride (MsCl, 1.2 mL, 15 mmol) was added dropwise to a 50 mL DMF solution containing **S7** (2 g, 4.89 mmol), LiCl (0.64 g, 15 mmol) and 2,4,6-collidine (2.6 mL, 20 mmol) at -3°C. The reaction mixture was stirred for 2 h and was then poured into ice-cold saturated NaHCO₃ solution (50 mL) and extracted with 1:1 hexane/Et₂O (3 × 50 mL). The combined organic phases were washed with saturated NH₄Cl, brine and dried over anhydrous Na₂SO₄ (EtOAc: petroleum ether = 15:85, R_{f}= 0.68). Evaporation followed by silica gel column chromatography gave (2*Z*, 6*Z*)-1-(*tert-*butyldiphenylsilyloxy)-8-chloro-3,7-dimethylocta-2,6-diene (12) as colourless syrup (1.7 g, 80%).

### Reference Example 4: (2Z,6Z,10Z,14Z)-1-(tert-Butyldiphenylsilyloxy)-16-chloro-3,7,11,15-tetramethylocta-2,6-diene (13)

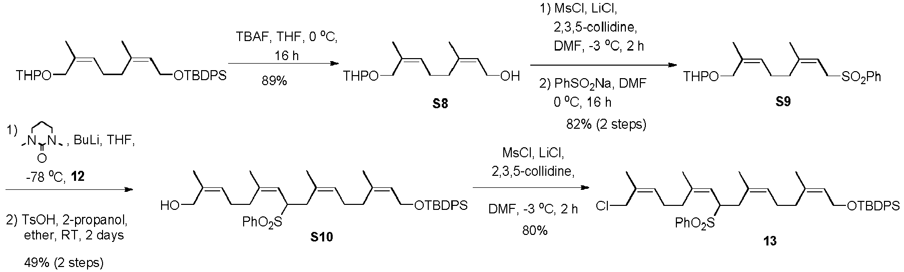

**S6** (4g, 8.11 mmol) was dissolved in 20 mL of THF, to which a solution of tetrabutylammonium fluoride (TBAF, 75%, 2.83 g, 8.11 mmol) in THF (10 mL) was added. The reaction mixture was stirred at 0°C for 16 h and was then diluted with petroleum ether (200 mL) and washed with brine (3 × 50 mL). The organic phase was dried with anhydrous Na₂SO₄ and concentrated to give crude (2*Z*, 6*Z*)-3,7-dimethyl-8-(2-tetrahydropyranyl)-octa-2,6-dien-1,8-diol (**S8**) as yellowish syrup. Silica gel column chromatography (EtOAc: dichloromethane = 1:9, R_{f}= 0.2) gave pure **S8** as colourless syrup.

Methanesulfonyl chloride (MsCl, 1.7 mL, 22 mmol) was added dropwise to a 50 mL DMF solution containing **S8** (1.8 g, 7.08 mmol), LiCl (0.907 g, 21.4 mmol) and 2,4,6-collidine (3.8 mL, 28.5 mmol) at -3°C. The reaction mixture was warmed up to 0°C in 2 h and then poured into ice-cold saturated NaHCO₃ solution (50 mL) and extracted with 1:1 hexane/Et₂O (3 × 50 mL). The combined organic phases were washed with saturated NH₄Cl and brine, and dried over anhydrous Na₂SO₄, filtered and concentrated. The crude product was used in the following reaction without further purification.

The crude chlorinate compound was then added NaSO₂Ph (2.5 g, 15.4 mmol) and stirred at RT for 16 h before diluted with EtOAc, washed with 1 M HCl, NaHCO₃, water, brine and dried over Na₂SO₄ which was then purified by silica gel column chromatography (EtOAc: petroleum ether = 15:85, R_{f}=0.16) to give (2*Z*, 6*Z*)-1-benzenesulfonyl-3,7-dimethyl-8-(2-tetrahydropyranyloxy)-octa-2,6-dienel (**S9**) as a colourless oil (1.6 g, 82%).

BuLi (1.4 M, 2.43 mL, 3.4 mmol) was added dropwise to a THF solution (10 mL) containing **S9** (660 mg, 1.7 mmol) and 1,3-dimethyl-3,4,5,6-tetrahydro-2(1*H*)-pyrimidinone (DMTP, 0.6 mL, 5.4 mmol) at -78 °C to give an orange coloured solution. After 2 h, a solution of **3a** (720 mg, 1.7 mmol) in THF (10 mL) was added dropwise over 90 min. The reaction mixture was then warmed up to -30 0°C and kept stirred at this temperature for 2 h. The reaction was quenched by pouring into saturated NH₄Cl (20 mL). The aqueous phase was extracted with 1:1 hexane/Et₂O (3 × 50 mL). The combined organic phase was washed with brine (3 × 50 mL) and dried over anhydrous Na₂SO₄. Solvent was removed *in vacuo* to give a yellow oil, which was then purified by silica gel column chromatography (EtOAc: petroleum ether = 15:85, R_{f}=0.22) to give a colourless oil.

The coupled product was dissolved in 2-propanol/Et₂O (20 mL/10 mL), to which was added *p*-toluene sulfonic acid (20 mg), the reaction was stirred at RT for 4 days, and was then diluted with Et₂O (250 mL), extracted with saturated NaHCO₃ solution, brine and dried over anhydrous Na₂SO₄. Evaporation followed by silica gel column chromatography (EtOAc: petroleum ether = 15:85, R_{f}= 0.11) gave (2*Z*,6*Z*,10*Z*, 14*Z*)-1-*O*-(*tert-*butyldiphenylsilyl)-3,7,11,15-tetramethyLocta-2,6-dien-1,16-diol (**S10**) as colourless syrup (590 mg, 49%).

Methanesulfonyl chloride (MsCl, 1.2 mL, 15 mmol) was added dropwise to a 10 mL DMF solution containing **S10** (440 mg, 0.584 mmol), LiCl (75 mg, 1.75 mmol) and 2,4,6-collidine (308 µL, 2.34 mmol) at -3°C. The reaction mixture was stirred for 2 h and was then poured into ice-cold saturated NaHCO₃ solution (50 mL) and extracted with 1:1 hexane/Et₂O (3 × 50 mL). The combined organic phases were washed with saturated NH₄Cl, brine and dried over anhydrous Na₂SO₄ (EtOAc: petroleum ether = 15:85, R_{f}= 0.18). Evaporation followed by silica gel column chromatography gave pure (2*Z*,6*Z*,10*Z*, 14*Z*)-1-(*tert*-butyldiphenylsilyloxy)-16-chloro-3,7,11,15-tetramethylocta-2,6-diene (**13**) as colourless syrup (263 mg, 80%).

### Reference Example 5: (2Z,6Z,10Z)-1-(tert-Butyldiphenylsilyloxy)-9-benzenesulfonyl-3,7,11,15-tetramethylhexadeca-2,6,10,14-tetraene (15)

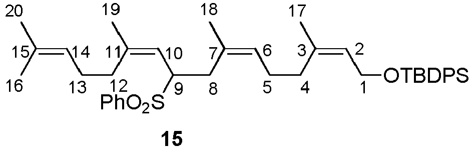

BuLi (1.4 M, 610 µL, 0.854 mmol) was added dropwise to a THF solution (2.5 mL) containing nerylsulfone (**9**, 250 mg, 0.9 mmol) and 1,3-dimethyl-3,4,5,6-tetrahydro-2(1*H*)-pyrimidinone (DMTP, 0.5 mL) at -78 °C to give an orange coloured solution. After 2 h, a solution of **12** (475 mg, 1.1 mmol) in THF (10 mL) was added dropwise over 90 min. The reaction mixture was then warmed up to -30 °C in 2h and kept stirred at this temperature for 2 h. The reaction was quenched by pouring into saturated NH₄Cl (20 mL). The aqueous phase was extracted with 1:1 hexane/Et₂O (3 × 50 mL). The combined organic phase was washed with brine (3 × 50 mL) and dried over anhydrous Na₂SO₄. Solvent was removed *in vacuo* to give a yellow oil, which was then purified by silica gel column chromatography (1:1 petroleum ether: DCM, R_{f}=0.12) to give **15** as colourless oil (379 mg, 63%).

### Reference Example 6: (2Z,6Z,10Z)-3,7,11,15-tetramethylhexadeca-2,6,10,14-tetraen-1-ol (3)

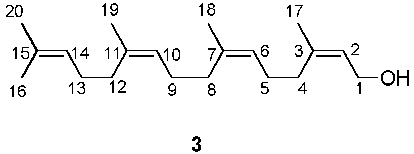

Sulfone **15** (100mg, 0.15 mmol) was dissolved in 20 mL of THF, to which a solution of 1M tetrabutylammonium fluoride (TBAF, 0.3 mL, 0.3 mmol) in THF was added. The reaction mixture was stirred at 0°C for 16 h and was then diluted with diethyl ether (200 mL) and washed with brine (3 × 50 mL). The organic phase was dried with anhydrous Na₂SO₄ and concentrated to give a yellowish syrup. The product was used in the next step without further purification.

1M Super-Hydride solution (LiEt₃BH, 0.75 mL, 0.75 mmol) was added dropwise to a 5 mL THF solution containing the sulponyl alcohol (0.15 mmol) and bis(diphenylphosphino) propanepalladium(II) dichloride ((dppp)PdCl₂, 29 mg, 50 µmol) at 0°C and the solution turned dark brown. The reaction mixture was stirred at 0°C for 2 h.¹¹

Saturated NH₄Cl solution (10 mL) was then added and the mixture was extracted with Et₂O (3 × 20 mL). The combined organic extracts were washed with brine (3 × 20 mL), dried over anhydrous Mg₂SO₄, and concentrated to give crude nerylnerol (**3**) as dark syrup. The crude compound was purified using silica gel column chromatography (DCM, R_{f}= 0.36) to give a colourless oil (31 mg, 70%).

### Reference Example 7: (2Z,6Z,10E)-1-(tert-Butyldiphenylsilyloxy)-9-benzenesulfonyl-3,7,11,15-tetramethylhexadeca-2,6,10,14-tetraene (16)

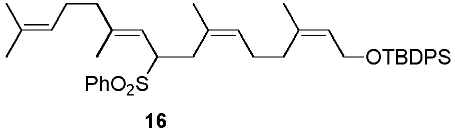

BuLi (1.5 M, 360 µL, 0.854 mmol) was added dropwise to a THF solution (3 mL) containing geranylsulfone (**10**, 150 mg, 0.54 mmol) and 1,3-dimethyl-3,4,5,6-tetrahydro-2(1*H*)-pyrimidinone (DMTP, 0.5 mL) at -78 °C to give an orange coloured solution. After 2 h, a solution of **12** (115 mg, 0.27 mmol) in THF (10 mL) was added dropwise over 90 min. The reaction mixture was then warmed up to -30 °C in 6h and kept stirred at this temperature for 2 h. The reaction was quenched by pouring into saturated NH₄Cl (20 mL). The aqueous phase was extracted with 1:1 hexane/Et₂O (3 × 50 mL). The combined organic phase was washed with brine (3 × 50 mL) and dried over anhydrous Na₂SO₄. Solvent was removed *in vacuo* to give a yellow oil, which was then purified by silica gel column chromatography (1:1 petroleum ether: DCM, R_{f}=0.12) to give **16** as colourless oil (120 mg, 67%).

### Reference Example 8: (2Z,6Z,10E)-3,7,11,15-tetramethylhexadeca-2,6,10,14-tetraen-1-ol (4)

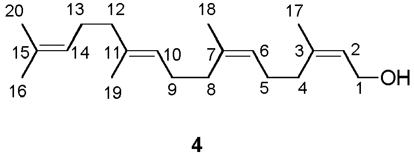

Sulfone **16** (72mg, 0.168 mmol) was dissolved in 20 mL of THF, to which a solution of 1M tetrabutylammonium fluoride (TBAF, 0.3 mL, 0.3 mmol) in THF was added. The reaction mixture was stirred at 0°C for 16 h and then diluted with diethyl ether (200 mL) and washed with brine (3 × 50 mL). The organic phase was dried with anhydrous Na₂SO₄ and concentrated to give a yellowish syrup. The product was used in the next step without further purification.

1M Super-Hydride solution (LiEt₃BH, 0.84 mL, 0.84 mmol) was added dropwise to a 5 mL THF solution containing the sulponyl alcohol (0.168 mmol) and bis(diphenylphosphino) propanepalladium(II) dichloride ((dppp)PdCl₂, 30 mg, 55 µmol) at 0°C and the solution turned dark brown. The reaction mixture was stirred at 0°C for 2 h.¹¹ Saturated NH₄Cl solution (10 mL) was then added and the mixture was extracted with Et₂O (3 × 20 mL). The combined organic extracts were washed with brine (3 × 20 mL), dried over anhydrous Mg₂SO₄, and concentrated to give crude geranylnerol (**1e**) as dark syrup. The crude compound was purified using silica gel column chromatography (DCM, R_{f}= 0.36) to give a colourless oil (29 mg, 60%).

### Reference Example 9: -(2Z,6Z,10Z,14Z,18Z)-1-(tert-Butyldiphenylsilyloxy)-9,17 di(benzenesulfonyl)-3,7,11,15,19,23-hexamethyltetraicosa-2,6,10,14,18,22-hexaene (17)

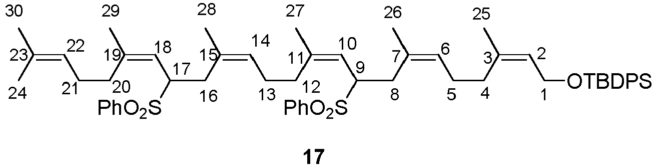

BuLi (1.5 M, 0.14 µL, 0.21 mmol) was added dropwise to a THF solution (0.5 mL) containing nerylsulfone (**9**, 40 mg, 0.142 mmol) and 1,3-dimethyl-3,4,5,6-tetrahydro-2(1*H*)-pyrimidinone (DMTP, 0.1 mL) at -78 °C to give an orange coloured solution. After 2 h, a solution of **13** (50 mg, 0.071 mmol) in THF (0.5 mL) was added dropwise over 90 min. The reaction mixture was then warmed up to -30 °C in 6 h and kept stirred at this temperature for 2 h. The reaction was quenched by pouring into saturated NH₄Cl (20 mL). The aqueous phase was extracted with 1:1 hexane/Et₂O (3 × 50 mL). The combined organic phase was washed with brine (3 × 50 mL) and dried over anhydrous Na₂SO₄. Solvent was removed *in vacuo* to give a yellow oil, which was then purified by silica gel column chromatography (DCM, R_{f}=0.38) to give **17** as colourless oil (62 mg, 93%).

### Reference Example 10: (2Z,6Z,10Z,14Z,18Z)-3,7,11,15,19,23-hexamethyltetraicosa-2,6,10,14,18,22-hexaen-1-ol (5)

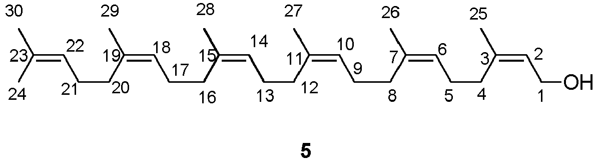

Sulfone **17** (20mg, 0.0207 mmol) was dissolved in 2 mL of THF, to which a solution of 1M tetrabutylammonium fluoride (TBAF, 0.05 mL, 0.05 mmol) in THF was added. The reaction mixture was stirred at 0°C for 16 h and was then diluted with diethyl ether (25 mL) and washed with brine (3 × 10 mL). The organic phase was dried with anhydrous Na₂SO₄ and concentrated to give a yellowish syrup. The product was used in the next step without further purification.

1M Super-Hydride solution (LiEt₃BH, 0.2 mL, 0.2 mmol) was added dropwise to a 1 mL THF solution containing the sulphonyl alcohol (0.02 mmol) and bis(diphenylphosphino) propanepalladium(II) dichloride ((dppp)PdCl₂, 5 mg, 9 µmol) at 0°C and the solution turned dark brown. The reaction mixture was stirred at 0°C for 3 h.¹¹ Saturated NH₄Cl solution (10 mL) was then added and the mixture was extracted with Et₂O (3 × 20 mL). The combined organic extracts were washed with brine (3 × 20 mL), dried over anhydrous Mg₂SO₄, and concentrated to give crude prenol **5** as dark syrup. The crude compound was purified using silica gel column chromatography (DCM, R_{f}= 0.23) to give a colourless oil (5 mg, 57%).

### Reference Example 11: -(2Z,6Z,10Z,14Z,18E)-1-(tert-Butyldiphenylsilyloxy)-9,17 di(benzenesulfonyl)-3,7,11,15,19,23-hexamethyltetraicosa-2,6,10,14,18,22-hexaene (18)

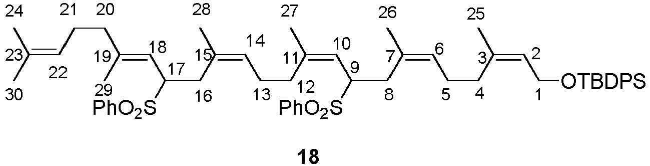

BuLi (1.5 M, 0.14 µL, 0.21 mmol) was added dropwise to a THF solution (0.5 mL) containing geranylsulfone (**10**, 40 mg, 0.142 mmol) and 1,3-dimethyl-3,4,5,6-tetrahydro-2(1*H*)-pyrimidinone (DMTP, 0.1 mL) at -78 °C to give an orange coloured solution. After 2 h, a solution of **13** (50 mg, 0.071 mmol) in THF (0.5 mL) was added dropwise over 90 min. The reaction mixture was then warmed up to -30 °C in 6h and kept stirred at this temperature for 2 h. The reaction was quenched by pouring into saturated NH₄Cl (20 mL). The aqueous phase was extracted with 1:1 hexane/Et₂O (3 × 50 mL). The combined organic phase was washed with brine (3 × 50 mL) and dried over anhydrous Na₂SO₄. Solvent was removed *in vacuo* to give a yellow oil, which was then purified by silica gel column chromatography (DCM, R_{f}=0.38) to give **18** as colourless oil (56 mg, 83%).

### Reference Example 12: (2Z,6Z,10Z,14Z,18E)-3,7,11,15,19,23-hexamethyltetraicosa-2,6,10,14,18,22-hexaen-1-ol (6)

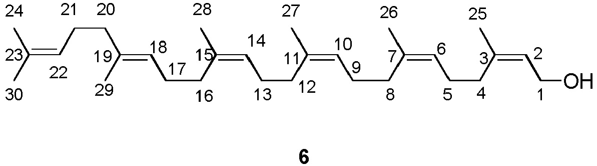

Sulfone **18** (45 mg, 0.0476 mmol) was dissolved in 2 mL of THF, to which a solution of 1M tetrabutylammonium fluoride (TBAF, 0.05 mL, 0.05 mmol) in THF was added. The reaction mixture was stirred at 0°C for 16 h and was then diluted with diethyl ether (25 mL) and washed with brine (3 × 10 mL). The organic phase was dried with anhydrous Na₂SO₄ and concentrated to give a yellowish syrup. The product was used in the next step without further purification.

1M Super-Hydride solution (LiEt₃BH, 0.45 mL, 0.45 mmol) was added dropwise to a 1 mL THF solution containing the sulphonyl alcohol (0.0476 mmol) and bis(diphenylphosphino) propanepalladium(II) dichloride ((dppp)PdCl₂, 9 mg, 15 µmol) at 0°C and the solution turned dark brown. The reaction mixture was stirred at 0°C for 3 h.¹¹ Saturated NH₄Cl solution (10 mL) was then added and the mixture was extracted with Et₂O (3 × 20 mL). The combined organic extracts were washed with brine (3 × 20 mL), dried over anhydrous Mg₂SO₄, and concentrated to give crude prenol as dark syrup. The crude compound was purified using silica gel column chromatography (DCM, R_{f}= 0.23) to give **6** as a colourless oil (10 mg, 50%).

### Synthesis of compound 27:

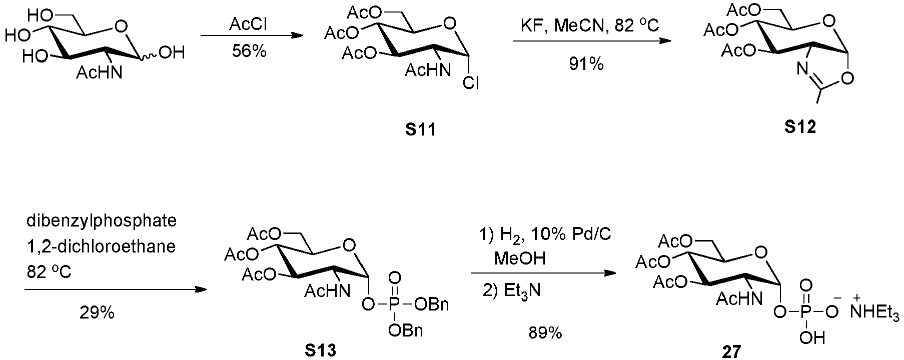

### Reference Example 13: 2-Acetylamido-3,4,6-tri-O-acetyl-2-deoxy-α-D-glucopyranosyl chloride (S11)

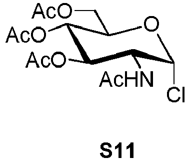

Acetyl chloride (10 mL, 141 mmol) was added through an air condenser into a round bottom flask containing 2-acetamido-2-deoxy-D-glucose (5 g, 22.6 mmol). The reaction mixture was stirred at room temperature for 48 h. TLC (petrol:ethyl acetate, 1:2) indicated formation of a product (Rf 0.3) with complete consumption of the starting material (Rf 0). The reaction mixture was diluted with DCM (150 mL) and then poured on to ice water (100 mL). The organic layer was washed with ice cold NaHCO₃ (3 x 100 mL) until no more gas was evolved. The organic layer was dried over MgSO₄, filtered and concentrated in vacuo. Purification using Silica gel column chromatography gave 2-acetamido-3,4,6-tri*O*-acetyl-2-deoxy-α-D-glucopyranosyl chloride (**S11**) as a colourless solid (4.65 g, 56%).

### Reference Example 14: 2-Methyl-(3,4,6-tri-O-acetyl-1,2-dideoxy-α-D-glucopyrano)[1,2-d]-oxazoline (S12)

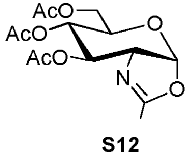

**S11** (1 g, 2.73 mmol) was added KF (0.65 g, 11.2 mmol), the two solids were left on the vacuum pump to dry for 1 h. MeCN (anhydrous, 10 mL) was added and the reaction mixture was refluxed at 82 °C for 16 h. Insolubles were filtered off and solvent was evaporated to give dark brown foam, which was purified by Silica gel column chromatography (EtOAc: petroleum ether = 4:1, R_{f}=0.125) to give 2-Methyl-(3,4,6-tri-*O-*acetyl-1,2-dideoxy-α-D-glucopyrano)[1,2-*d*]-oxazoline (**S12**) as a yellow solid (0.82 g, 91%).

### Reference Example 15: 2-Acetamido-3,4,6-tri-O-acetyl-2-deoxy-α-D-glucopyranose 1-dibenzylphosphate (S13)

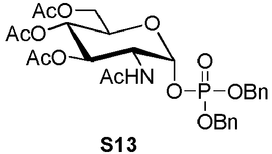

**S12** (1 g, 3 mmol) was dissolved in anhydrous 1,2-dichloroethane (10 mL) and stirred under an atmosphere of argon. A solution of dibenzyl phosphate (0.94 g, 3.3 mmol) in 1,2-dichloroethane (8 mL) was added dropwise to the sugar solution. After 24 h, the reaction was concentrated *in vacuo* and the resulting residue purified by flash column chromatography (diethyl ether:methanol, 95:5, Rf 0.38) to afford 2-acetamido-3,4,6-tri-*O-*acetyl-2-deoxy-α-D-glucopyranose 1-dibenzylphosphate (**S13**) as a yellow solid (510 mg, 29%).

### Reference Example 16: 2-Acetamido-3,4,6-tri-O-acetyl-2-deoxy-α-D-glucopyranose 1-phosphate (27)

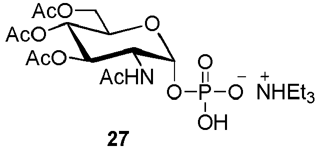

**S13** (100 mg) and 10% Pd/C (10 mg) was vacuumed on pump and re-charged with H₂ while flask was placed on dry ice. Anhydrous methanol (5 mL) was added. The reaction system was vacuumed and charged with hydrogen for three times. Reaction mixture was then stirred at room temperature under an atmosphere of hydrogen. After 4 h MS showed that starting material (m/z 630, +ve) had disappeared and turned into fully unprotected phosphate (m/z 426, -ve). The reaction mixture was then filtered through celite and triethylamine (25 µL). Solvent was removed *in vacuo* to give 2-acetamido-3,4,6-tri-*O-*acetyl-2-deoxy-α-D-glucopyranose 1-phosphate **(27)** as white solid (77.3 mg, 89%).

### Synthesis of glycosylated lipids:

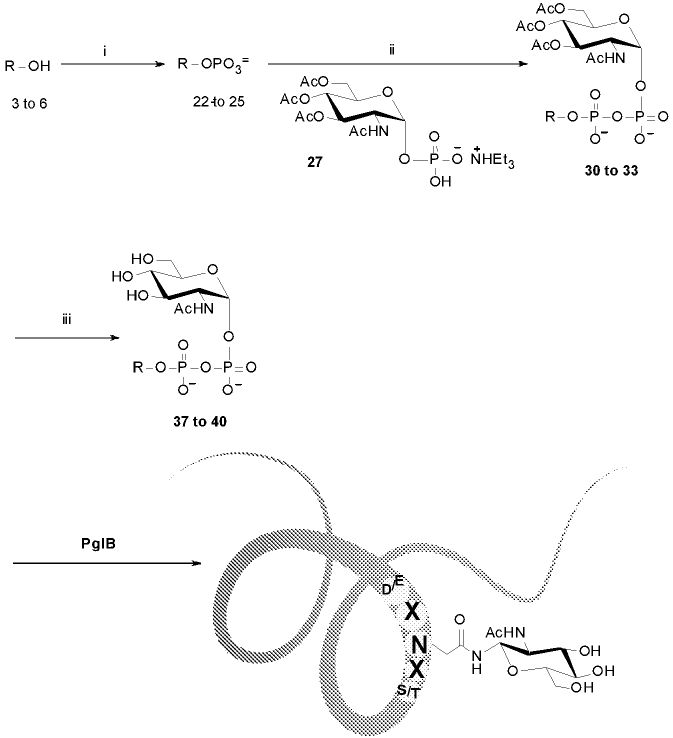

In this scheme, R represents the polyisoprenyl chain

### Example 1

### Step 1: (2Z,6Z,10Z)-3,7,11,15-tetramethylhexadeca-2,6,10,14-tetraen-1-yl phosphate (22, nerylneryl phosphate)

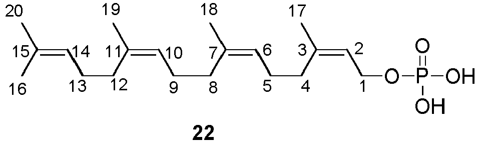

Trichloroacetonitrile (TCA, 42 µL, 0.4 mmol) was added to a stirred solution of prenol **3** (18 mg, 0.062 mmol) and tetra-n-butylammonium dihydrogen phosphate (106 mg, 0.3 mmol) in DCM (2 mL). The reaction mixture was stirred at room temperature for 10 min before solvent was removed *in vacuo.* The residue was re-dissolved in THF (1 mL) and 0.2 mL of 25% ammonium hydroxide solution in water was added. After 30 min, 5 mL 1:1 toluene:methanol was added and precipitate was removed by filtration after 20 min. After removal of solvent in *vacuo,* the residue was washed with petroleum ether (5 mL 3), dissolved in methanol (2 mL) and filtrated. Excessive Dowex 50WX8 (NH₄⁺form) was added and allowed to stir for 30 min before filtration. Methanol was removed and the residual solid was purified using silica gel column chromatography (water:isopropanol:ethylacetate = 1:2:4 R_{f}= 0.42) to give ammonium nerylnerylphosphate 22 as a white solid (14 mg, 56%).

### Step 2: P¹-2-Acetamido-3,4,6-tri-O-acetyl-2-deoxy-α-D-glucopyranosyl P²-(2Z,6Z,10Z)-3,7,11,15-tetramethylhexadeca-2,6,10,14-tetraen-1-yl diphosphate (30)

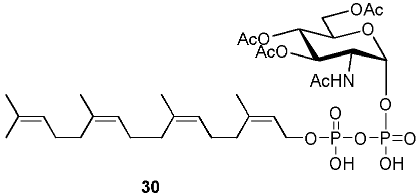

Nerylnerylphosphate (**22**, 14 mg, 0.035 mmol) and 1,1'-carbonyldiimidazole (CDI, 30 mg, 0.185 mmol) was dissolved in DMF (1 mL) and stirred at RT for 2 h. Anhydrous methanol (6 µL) was added and the reaction was stirred for an additional 45 min. Excessive methanol was then removed *in vacuo.* A solution of 2-acetamido-3,4,6,-tri-*O*-acetyl-2-deoxy-α-D-glucopyranose 1-phosphate (**27**, 75 mg) in DMF (1 mL) was added and the reaction was stirred for three days. DMF was removed *in vacuo* and compound **30** was purified using silica gel column chromatography (water:isopropanol:ethylacetate = 1:2:4 R_{f}= 0.29) to give triethylammonium/ammonium salt of **30** (0.6Et₃N.1.4NH₃) as a white solid (10 mg, 34 %).

### Step 3: P¹-2-Acetamido-2-deoxy-α-D-glucopyranosyl P²-(2Z,6Z,10Z)-3,7,11,15-tetramethyl hexadeca -2,6,10,14-tetraen-1-yl diphosphate (37; Example 1)

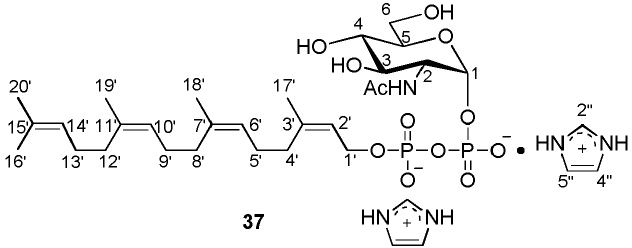

Compound **30** (5 mg, 6 µmol) was dissolved in 1 mL of anhydrous methanol to which 120 µL of freshly prepared 50 mM NaOMe in methanol solution was added. The reaction mixture was stirred at 0 °C for 1h and the NaOMe was quenched with Dowex 50WX8 (NH₄⁺form). After filtration and removal of methanol, reaction mixture was purified using silica gel column chromatography (water:isopropanol:ethylacetate = 1:2:4 R_{f}= 0.24). Conjugate **37 (Example 1)** was yielded as a white solid (3.6 mg, 88%). ¹H NMR (500 MHz, MeOD) δ 7.70 (s, 2H, 2×imdazole H-2"), 7.06 (s, 4H, 2×imdazole H-4" and 2×imdazole H-5"), 5.60 (dd, 1H, *J*_{1,P}6.6 Hz, *J*_{1,2} 2.9 Hz, H-1), 5.43 (t, 1H, *J*_{1',2'} 6.9 Hz, H-2'), 5.16 - 5.11 (m, 3H, H-6', H-10' and H-14'), 4.49 (dd, 2H, J_{1',2'} 7.0 Hz, *J*_{1',P} 7.0 Hz H-1'), 3.98 (dt, 1H *J*_{1,2} 3.0 Hz, *J*_{2,3} 10.6 Hz, H-2), 3.96 (m, 1H, H-5), 3.90 (d, 1H, *J*_{6a,6b} 11.6 Hz, H-6a), 3.76 (t, 1H, *J* 9.7 Hz, H-3), 3.68 (dd, 1H, *J*_{6a,6b} 11.9, *J*_{5,6b} 6.4 Hz, H-6b), 3.37 (dd, 1H, *J*_{2,3} 9.1 Hz, *J*_{3,4} 10 Hz, H-4), 2.20 - 1.97 (m, 16H), 2.06 (m, 5H), 1.77 (d, 3H, *J*_{2'}, _{17'} 1.3 Hz, H-17'), 1.70 (m, 9H, H-18', H-19', H-20'), 1.64 (s, 3H, H-16'). ¹³C NMR (126 MHz, MeOD) δ 141.29 (C-3'), 136.61(C-7'), 136.32(C-11' and 2×imidazole C-2"), 132.38(C-20'), 126.10, 125.75, 125.38 (C-6', C-10' and C-14'), 122.96(m, C-2'), 122.61(m, 2×imdazole C-4" and 2×imdazole C-5"), 96.2(C-1), 74.99(C-5), 72.57(C-3), 72.25(C-4), 63.79(d, *J*_{1',P} 5.2 Hz, C-1'), 62.97(C-6), 55.33(C-2), 33.29, 33.26(C-4' and C-8'), 32.89(C-12'), 27.73(C-13'), 27.59, 27.51(C-5' and C-9'), 25.96(C-20'), 23.75, 23.70×2 (C-17', C-18' and C-19'), 22.88(Ac), 17.77(C-16'). ³¹P(H) NMR (202 MHz, D₂O) δ -9.4, -11.6. ESI m/z 669.3 (M-2 imidzole+H⁺)⁻, calculated 669.2923 for C₂₈H₅₁N₂O₁₂P₂.

### Example 2:

### P¹-2-Acetamido-2-deoxy-α-D-glucopyranosyl P²-(2Z,6Z,10E)-3,7,11,15-tetramethyl hexadeca-2,6,10,14-tetraen-1-yl diphosphate (38)

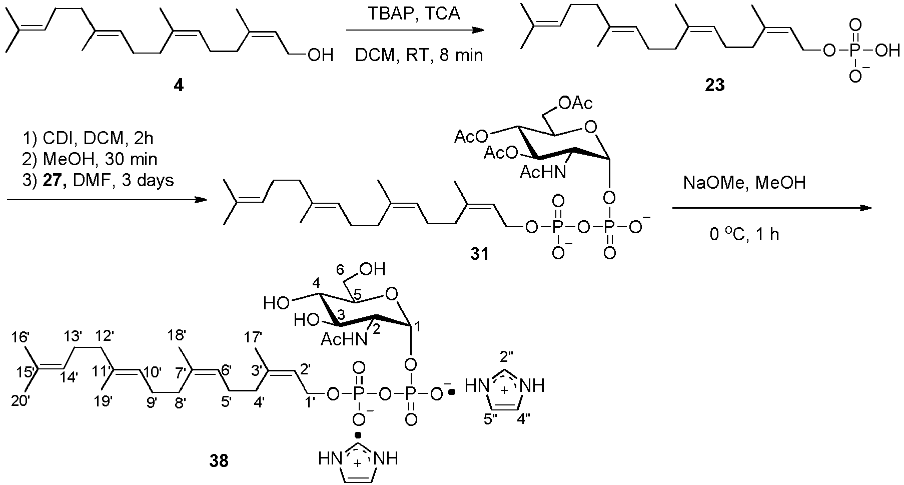

TCA (38 µL, 0.4 mmol) was added to a stirred solution of prenol **4** (22 mg, 0.076 mmol) and tetra-n-butylammonium dihydrogen phosphate (103 mg, 0.3 mmol) in DCM (1 mL). The reaction mixture was stirred at room temperature for 8 min before solvent was removed *in vacuo.* The residue was re-dissolved in THF (1 mL) and 0.2 mL of 25% ammonium hydroxide solution in water was added. After 30 min, 5 mL 1:1 toluene:methanol was added and precipitate was removed by filtration after 20 min. After removal of solvent in *vacuo,* the residue was washed with petroleum ether (5 mL×3), dissolved in methanol (2 mL) and filtrated. Excessive Dowex 50WX8 (NH₄⁺form) was added and allowed to stir for 30 min before filtration. Methanol was removed to give lipidphosphate **23** as a white solid (water:isopropanol:ethylacetate = 1:2:4 R_{f}= 0.36), which was used in the following reaction without further purification.

Lipid phosphate **23** and CDI (81.07mg, 0.5 mmol) were dissolved in DCM (0.5 mL) and stirred at RT for 2h. A solution of 5% v/v anhydrous methanol in DCM (0.364 mL) was added to quench unreacted CDI and stirred for 30 min. The solvents were then removed *in vacuo.* DMF (0.5 mL) and **27** (106 mg) were then added and the reaction mixture was stirred at RT for 5 days. DMF was then removed *in vacuo* and silica gel column chromatography (water:isopropanol:ethylacetate = 1:2:4 R_{f}= 0.22) gave crude imidazolium salt of **31** as a white solid.

Compound **31** was dissolved in 1 mL of anhydrous methanol to which 80 µL of freshly prepared 50 mM NaOMe in methanol solution was added. The reaction mixture was stirred at 0 °C for 1h and the NaOMe was quenched with Dowex 50WX8 (NH₄⁺form). After filtration and removal of methanol, reaction mixture was purified using silica gel column chromatography (water:isopropanol:ethylacetate = 1:2:4 R_{f}= 0.26). *P*¹-2-acetamido-2-deoxy-α-D-glucopyranosyl *P*²-(2*Z*,6*Z*,10*E*)-3,7,11,15-tetramethyl hexadeca-2,6,10,14-tetraen-1-yl diphosphate (**38; Example 2**) was yielded as a white solid (4.6 mg, 9% over 3 steps). ¹H NMR (500 MHz, MeOD) δ 7.71 (s, 2H, 2×imdazole H-2"), 7.07 (s, 4H, 2×imdazole H-4" and 2×imdazole H-5"), 5.61 (dd, 1H, *J*_{1,P} 6.6 Hz, *J*_{1,2} 2.9 Hz, H-1), 5.43 (t, 1H, J_{1',2'} 6.5 Hz, H-2'), 5.16 - 5.11 (m, 2H, H-6' and H-10'), 5.09 (tqq, 1H, *J*_{13',14'} 7.2 Hz, *J*_{14',16'} 1.0 Hz, *J*_{14',20'} 1.0 Hz, H-14') 4.49 (dd, 2H, *J*_{1',2'} 7.0 Hz, *J*_{1',P} 7.0 Hz, H-1'), 3.98 (dt, ¹H *J*_{1,2} 3.0 Hz, *J*_{2,3} 10.6 Hz, H-2), 3.96 (m, 1H, H-5), 3.90 (d, 1H, *J*_{6a,6b} 11.6 Hz, H-6a), 3.76 (t, 1H, *J* 9.7 Hz, H-3), 3.67 (dd, 1H, *J*_{6a,6b} 11.4, *J*_{5,6b} 6.4 Hz, H-6b), 3.37 (dd, 1H, *J*_{2,3} 9.1 Hz, *J*_{3,4} 10 Hz, H-4), 2.16 - 2.04 (m, 10H, H-4', H-5', H-8', H-9', H-13'), 2.03 (s, 3H, NHCOCH₃), 1.98 (m, 2H, H-12') 1.77 (d, 3H, *J*_{2'}, _{17'} 1.3 Hz, H-17'), 1.70 (m, 9H, H-18', H-19', H-20'), 1.64 (s, 3H, H-16'). ¹³C NMR (126 MHz, MeOD) δ 141.37 (C-3'), 136.75(C-7'), 136.33(2×imidazole C-2"), 136.17(C-11'), 132.12(C-15'), 125.67, 125.41, 125.35(C-6', C-10' and C-14'), 122.86(C-2'), 122.60(m, 2×imdazole C-4" and 2×imdazole C-5"), 96.2(C-1), 74.98(C-5), 72.48(C-3), 72.25(C-4), 63.77(d, *J*_{1',P} 4.8Hz, C-1'), 62.95(C-6), 55.29(d, *J*_{2,P} 5.2Hz, C-2), 40.89(C-12'), 33.30(C-4'), 32.89(C-8'), 27.75(C-13'), 27.58, 27.54(C-5' and C-9'), 25.90(C-20'), 23.72, 23.70(C-17' and C-18'), 22.86(NHCOCH₃), 17.77(C-16'), 16.09(C-19'). ³¹P(H) NMR (162 MHz, D₂O) δ -9.6, -11.6. ESI m/z 669.3 (M-2 imidzole+H⁺)⁻, calculated 669.2923 for C₂₈H₅₁N₂O₁₂P₂.

### Example 3:

### P¹-2-Acetamido-2-deoxy-α-D-glucopyranosyl P²-(2Z,6Z,10Z,14Z,18Z)-3,7,11,15,19, 23-hexamethyltetraicosa-2,6,10,14,18,22-hexaen-1-yl diphosphate (39)

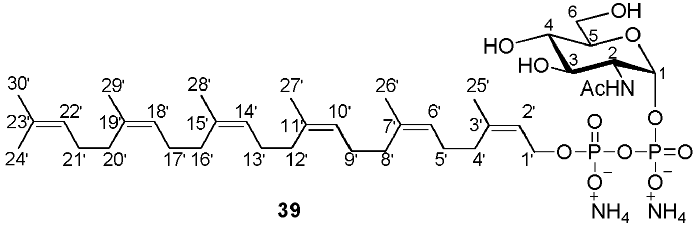

Trichloroacetonitrile (TCA, 16 µL, 0.156 mmol) was added to a stirred solution of prenol 5 (13.6 mg, 0.032 mmol) and tetra-n-butylammonium dihydrogen phosphate (43.3 mg, 0.128 mmol) in DCM (2 mL). The reaction mixture was stirred at room temperature for 10 min before solvent was removed *in vacuo.* The residue was re-dissolved in THF (1 mL) and 0.1 mL of 25% ammonium hydroxide solution in water was added. After 30 min, 5 mL 1:1 toluene:methanol was added and precipitate was removed by filtration after 20 min. After removal of solvent in *vacuo,* the residue was washed with petroleum ether (5 mL× 3), dissolved in methanol (2 mL) and filtrated. Excessive Dowex 50WX8 (NH₄⁺form) was added and allowed to stir for 30 min before filtration. Methanol was removed and the residue solid (phosphate **24**) was used in the next step without purification.

Prenylphosphate **24** (from the last step) and 1,1'-carbonyldiimidazole (CDI, 48.6 mg, 0.3 mmol) was dissolved in DMF (1.0 mL) and stirred at RT for 2 h. Anhydrous methanol (11 µL) was added and the reaction was stirred for an additional 45 min. Excessive methanol was then removed *in vacuo.* A solution of 2-acetamido-3,4,6,-tri-*O*-acetyl-2-deoxy-α-D-glucopyranose 1- phosphate (**6**, 64 mg, 0.15 mmol) in DMF (1.0 mL) was added and the reaction was stirred for three days. DMF was removed *in vacuo* and compound **32** was purified using silica gel column chromatography (water:isopropanol:ethylacetate = 1:2:4 R_{f}= 0.26) to give the ammonium salt of *P*¹-2-acetamido-3,4,6-tri-*O*-acetyl-2-deoxy-α-D-glucopyranosyl *P*²-(2*Z*,6*Z*,10*Z*, 14*Z*,18*Z*)-3,7,11,15,19,23-hexamethyltetraicosa-2,6,10,14,18,22-hexaen-1-yl diphosphate (**32**) as a white solid.

Compound **32** from the previous step was dissolved to 1 mL of anhydrous methanol to which 120 µL of freshly prepared 50 mM NaOMe in methanol solution was added. The reaction mixture was stirred at 0 °C for 1h and the NaOMe was quenched with Dowex 50WX8 (NH₄⁺ form). After filtration and removal of methanol, reaction mixture was purified using silica gel column chromatography (water:isopropanol:ethylacetate = 1:2:4 R_{f}= 0.15). *P*¹-2-Acetamido-2-deoxy-α-D-glucopyranosyl *P*²-(2*Z*,6*Z*,10*Z*,14*Z*,18*Z*)-3,7,11,15,19, 23-hexamethyltetraicosa-2,6,10,14,18,22-hexaen-1-yl diphosphate (**39; Example 3**) was yielded as a white solid (6 mg, 23% over 3 steps). ¹H NMR (500 MHz, MeOD) δ 5.60 (dd, 1H, *J*_{1,P} 6.6 Hz, *J*_{1,2} 2.9 Hz, H-1), 5.45 (t, 1H, *J*_{1',2'} 6.9 Hz, H-2'), 5.20 - 5.11 (m, 6H, H-5, H-6', H-10', H-14', H-18', H-22'), 4.51 (dd, 2H, J_{1',2'} 7.0 Hz, J_{1',P} 7.0 Hz, H-1'), 4.00 (dt, 1H *J*_{1,2} 3.0 Hz, *J*_{2,3} 10.6, H-2), 3.98 (m, 1H, H-5), 3.93 (d, 1H, *J*_{6a,6b} 11.6 Hz, H-6a), 3.76 (t, 1H, *J* 9.7 Hz, H-4), 3.68 (dd, 1H, *J*_{6a,6b} 11.9, *J*_{5,6b} 6.4 Hz, H-6b), 3.37 (dd, 1H, *J*_{2,3} 9.1 Hz, *J*_{3,4} 10 Hz, H-3), 2.20 - 1.97 (m, 20H, H-4', H-5', H-8', H-9', H-12', H-13', H-16', H-17', H-20', H-21'), 2.06 (s, 3H, NHCOCH₃), 1.77 (s, 3H, H-25), 1.70 (m, 15H, H-26', H-27', H-28', H-29', H-30'), 1.64 (s, 3H, H-24'); ³¹P(H) NMR (162 MHz, D₂O) δ -9.79, -11.73; HRMS m/z 805.4168 (M-NH₄)⁻, calculated 805.4175 for C₃₈H₆₇N₂O₁₂P₂.

### Example 4:

### P¹-2-Acetamido-2-deoxy-α-D-glucopyranosyl P²-(2Z,6Z,10Z,14Z,18E)-3,7,11,15,19,23-hexamethyltetraicosa-2,6,10,14,18,22-hexaen-1-yl diphosphate (40)

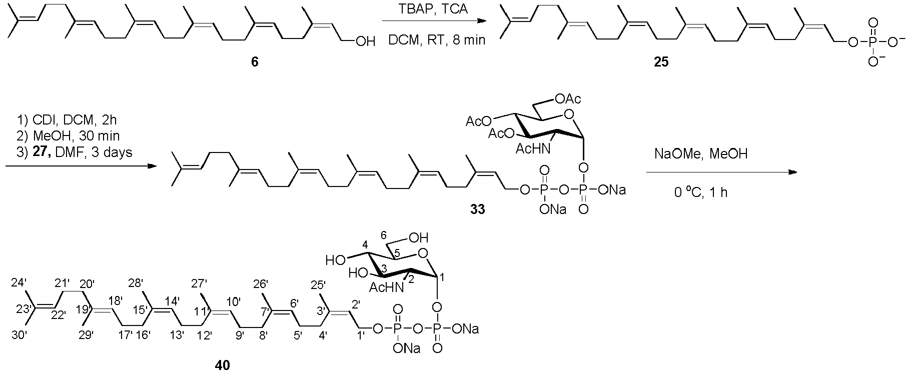

TCA (13.8 µL, 0.138 mmol) was added to a stirred solution of prenol 6 (11.8 mg, 0.028 mmol) and tetra-n-butylammonium dihydrogen phosphate (37.54 mg, 0.11 mmol) in DCM (1 mL). The reaction mixture was stirred at room temperature for 8 min before solvent was removed *in vacuo.* The residue was re-dissolved in THF (1 mL) and 0.2 mL of 25% ammonium hydroxide solution in water was added. After 30 min, 5 mL 1:1 toluene:methanol was added and precipitate was removed by filtration after 20 min. After removal of solvent in *vacuo,* the residue was washed with petroleum ether (5 mL×3), dissolved in methanol (2 mL) and filtrated. Excessive Dowex 50WX8 (NH₄⁺form) was added and allowed to stir for 30 min before filtration. Methanol was removed to give lipidphosphate **25** as a white solid (water:isopropanol:ethylacetate = 1:2:4 R_{f}= 0.40), which was used in the following reaction without further purification.

Lipid phosphate **25** and CDI (43.8mg, 0.27 mmol) were dissolved in DCM (0.5 mL) and stirred at RT for 2h. A solution of 5% v/v anhydrous methanol in DCM (0.196 mL) was added to quench unreacted CDI and stirred for 30 min. The solvents were then removed *in vacuo.* DMF (0.5 mL) and **27** (58 mg) were then added and the reaction mixture was stirred at RT for 5 days. DMF was then removed *in vacuo* and silica gel column chromatography (water:isopropanol:ethylacetate = 1:2:4 R_{f}= 0.26) to gave crude imidazoloum salt of 33 as a white solid.

Compound **33** was dissolved in 1 mL of anhydrous methanol to which 80 µL of freshly prepared 50 mM NaOMe in methanol solution was added. The reaction mixture was stirred at 0 °C for 1h and the NaOMe was quenched with Dowex 50WX8 (NH₄⁺form). After filtration and removal of methanol, reaction mixture was purified using silica gel column chromatography (water:isopropanol:ethylacetate = 1:2:4 R_{f}= 0.29). Conjugate **40 (Example 4)** was yielded as a white solid (2.0 mg, 9% over 3 steps). ¹H NMR (500 MHz, MeOD) δ 5.60 (dd, 1H, *J*_{1,P}6.6 Hz, *J*_{1,2} 2.9 Hz, H-1), 5.43 (t, 1H, *J*_{1',2'} 6.9 Hz, H-2'), 5.17 - 5.11 (m, 4H, H-6', H-10', H-14', H-18'), 5.08 (tqq, 1H, *J*_{21',22'}7.2 Hz, *J*_{22',24'} 1.0 Hz, *J*_{22',30'} 1.0 Hz, H-22'), 4.49 (dd, 2H, J_{1',2'} 7.0 Hz, *J*_{1',P} 7.0 Hz, H-1'), 3.99 (dt, 1H *J*_{1,2} 3.0 Hz, *J*_{2,3} 10.6, H-2), 3.97 (m, 1H, H-5), 3.91 (m, 1H, H-6a), 3.73 (t, 1H, *J*_{3,4} 9.7 Hz, H-3), 3.67 (dd, 1H, *J*_{6a,6b} 11.9, *J*_{5,6b} 6.4 Hz, H-6b), 3.37 (dd, 1H, *J*_{3,4} 10 Hz, *J*_{4,5} 9.1 Hz, H-4), 2.20 - 1.97 (m, 18H, H-4', H-5', H-8', H-9', H-12', H-13', H-16', H-17', H-21'), 2.04 (s, 3H, Ac), 1.98 (m, 2H, H-20') 1.74 (d, 3H, *J*_{2',25'} 1.0 Hz, H-25'), 1.69-1.67 (m, 9H, H-26', H-27', H-28'), 1.67 (d, 3H, *J*_{22',30'} 1.0 Hz, H-30'), 1.61 (d, 3H, *J*_{18',29'} 1.0 Hz, H-29'), 1.60 (s, 3H, H-24'). ¹³C NMR (126 MHz, MeOD) δ 141.27 (C-3'), 136.58, 136.35, 136.30, 136.14(C-7', C-11', C-15' and C-19'), 132.12(C-23'), 126.16, 126.14, 125.81, 125.44, 125.37(C-6', C-10', C-14', C-18' and C-22'), 122.99(m, C-2'), 96.2(C-1), 75.00(C-5), 72.56(C-3), 72.29(C-4), 63.80(d, *J*_{1',P} 4.8Hz, C-1'), 63.00(C-6), 55.34(d, *J*_{2,P} 5.2Hz, C-2), 40.91(C-20'), 33.30, 33.28, 33.27(C-4', C-8' and C-12'), 32.87(C-16'), 27.77(C-21'), 27.68, 27.62, 27.59, 27.55(C-5', C-9', C-13' and C-17'), 25.94(C-30'), 23.84, 23.80, 23.76, 23.74(C-25', C-26', C-27',and C-28'), 22.92(Ac), 17.82(C-24'), 16.16(C-29'). ³¹P(H) NMR (162 MHz, D₂O) δ -9.5, -11.5.

### Comparative Example 5: P¹-2-Acetamido-2-deoxy-α-D-glucopyranosyl P²-(2Z,6Z,10Z,14Z,18Z,22E,26E)-3,7,11,15,19,23,27,31-octamethyldotriaconta-2,6,10,14,18,22,26,30-octaen-1-yl diphosphate (8a)

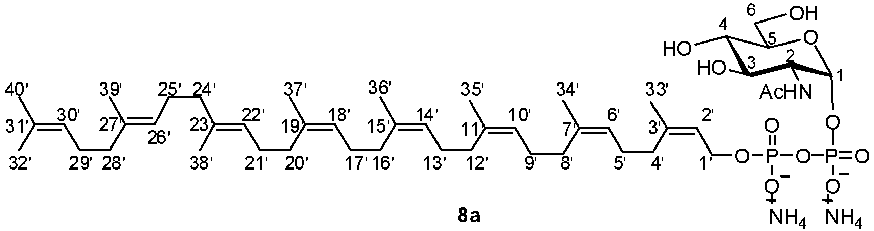

Trichloroacetonitrile (TCA, 8.9 µL, 0.089 mmol) was added to a stirred solution of prenol **1a** (10.0 mg, 0.0178 mmol) and tetra-n-butylammonium dihydrogen phosphate (24 mg, 0.071 mmol) in DCM (1 mL). The reaction mixture was stirred at room temperature for 10 min before solvent was removed *in vacuo.* The residue was re-dissolved in THF (1 mL) and 0.05 mL of 25% ammonium hydroxide solution in water was added. After 30 min, 5 mL 1:1 toluene:methanol was added and precipitate was removed by filtration after 20 min. After removal of solvent in *vacuo,* the residue was washed with petroleum ether (5 mL× 3), dissolved in methanol (2 mL) and filtrated. Excessive Dowex 50WX8 (NH₄⁺form) was added and allowed to stir for 30 min before filtration. Methanol was removed and the residue solid (phosphate **5a**) was used in the next step without further purification.

Prenylphosphate **5a** (from the last step) and 1,1'-carbonyldiimidazole (CDI, 27.6 mg, 0.55 mmol) was dissolved in DMF (1.0 mL) and stirred at RT for 2 h. Anhydrous methanol (6.2 µL) was added and the reaction was stirred for an additional 45 min. Excessive methanol was then removed *in vacuo.* A solution of 2-acetamido-3,4,6,-tri-O-acetyl-2-deoxy-α-D-glucopyranose 1- phosphate **6**, 36.3 mg, 077 mmol) in DMF (1.0 mL) was added and the reaction was stirred for three days. DMF was removed *in vacuo* and *P*¹-2-acetamido-3,4,6-tri-*O*-acetyl-2-deoxy-α-D-glucopyranosyl *P*²-(2*Z*,6*Z*,10*Z*,14*Z*,18*Z*,22*E*,26*E*)-3,7,11,15,19,23,27,31-octamethyldotriaconta-2,6,10,14,18,22,26,30-octaen-1-yl diphosphate (**7a**) was purified using silica gel column chromatography (water:isopropanol:ethylacetate = 1:2:4 R_{f}= 0.30) to give the ammonium salt as a white solid.

Compound **7a** was dissolved to 1 mL of anhydrous methanol to which 120 µL of freshly prepared 50 mM NaOMe in methanol solution was added. The reaction mixture was stirred at 0 °C for 1h and the NaOMe was quenched with Dowex 50WX8 (NH₄⁺ form). After filtration and removal of methanol, reaction mixture was purified using silica gel column chromatography (water:isopropanol:ethylacetate = 1:2:4 R_{f}= 0.18). *P*¹-2-acetamido-2-deoxy-α-D-glucopyranosyl *P*²-(2*Z*,6*Z*,10*Z*, 14*Z*,18*Z*,22*E*,26*E*)-3,7,11,15,19,23,27,31-octamethyldotriaconta-2,6,10,14,18,22,26,30-octaen-1-yl diphosphate (**8a; Comparative Example 5**) was yielded as a white solid (3 mg, 17% over 3 steps). ¹H NMR (500 MHz, MeOD) δ 5.60 (dd, 1H, *J*_{1,P}6.6 Hz, *J*_{1,2} 2.9 Hz, H-1), 5.45 (t, 1H, *J*_{1',2'} 6.9 Hz, H-2'), 5.20 - 5.11 (m, 7H, H-6', H-10', H-14', H-18', H-22', H-26', H-30'), 4.51 (dd, 2H, *J*_{1',2'} 7.0 Hz, *J*_{1',P} 7.0 Hz, H-1'), 4.00 (dt, 1H *J*_{1,2} 3.0 Hz, *J*_{2,3} 10.6, H-2), 3.98 (m, 1H, H-5), 3.93 (d, 1H, *J*_{6a,6b} 11.6 Hz, H-6a), 3.76 (t, 1H, *J* 9.7 Hz, H-4), 3.68 (dd, 1H, *J*_{6a,6b} 11.9, *J*_{5,6b} 6.4 Hz, H-6b), 3.37 (dd, 1H, *J*_{2,3} 9.1 Hz, *J*_{3,4} 10 Hz, H-3), 2.20 - 1.97 (m, 28 H, H-4', H-5', H-8', H-9', H-12', H-13', H-16', H-17', H-20', H-21', H-24', H-25', H-28', H-29'), 2.06 (s, 3H, NHCOCH₃), 1.77 (s, 3H, H-33), 1.69-1.64 (m, 15H, H-40', H-34', H-35', H-36', H-37), 1.64 (m, 9H, H-32', H-38', H-39'). HRMS *m*/*z* 941.5421 (M-NH₄)⁻, calculated 941.5427 for C₄₈H₈₃N₂O₁₂P₂.

### Example 6: PglB-catalyzed Glycosylation

### C. jejuni PglB Preparation and Purification

Recombinant *C. jejuni* PglB-10His was purified to near homogeneity from *E*. *coli* Rosetta as described by Gerber et al. (J. Biol Chem 2013, 288, 8849-61).

### In vitro analysis

A 50 µl reaction was prepared by mixing PglB (0.44 µM), peptide acceptor Tamra-DANYTK (e.g obtained from Invitrogen) (20 µM) and a synthetic lipid-linked carrier corresponding to one of Examples 1 to 4 (20 µM) in a reaction buffer (HEPES 10 mM, MnCl₂ 1 mM and 1% Triton-X 100, pH 7.5). This was incubated for 16 h at 26°C to obtain maximum glycosylation yield.

The reaction was also carried out with the compound of Comparative Example 5 and two further synthetic lipid-linked carriers corresponding to those of Examples 1 to 4 but having a shorter lipid chain of two isoprenyl units (nerol, Ref. A) and three isoprenyl units (cis-cis-farnesol, Ref. B) respectively. Glycosylation was also carried out with citronellol-pyrophospate-GlcNAc (Ref C) with controls (negative control= no lipid; positive control= using lipid extracted and enriched from *E.coli* cells producing *C.jejuni* heptasaccharide linked undecaprenyl pyrophosphate).

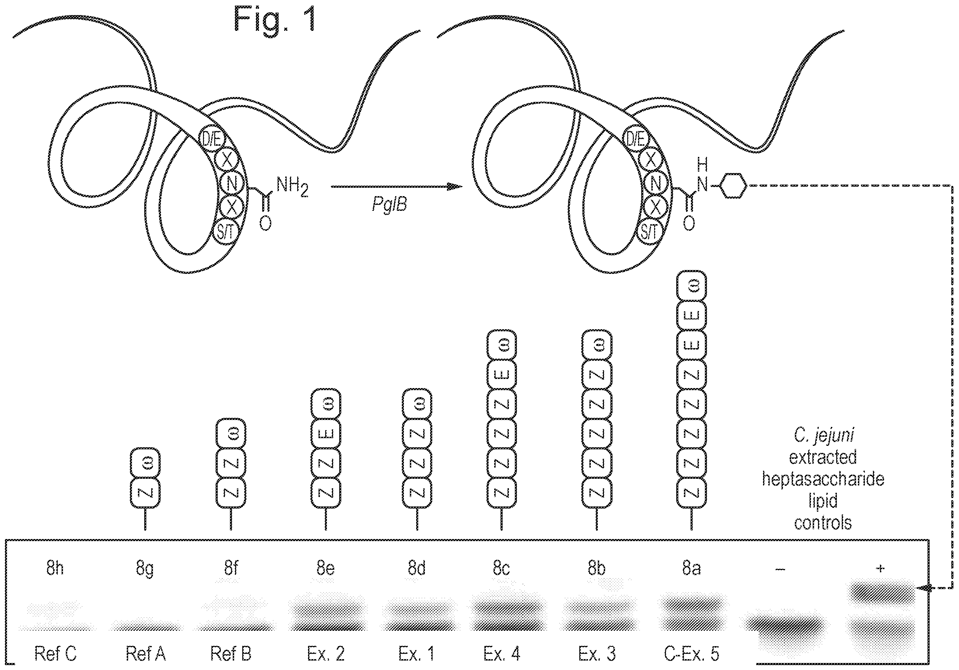

The glycosylation of the TAMRA-peptide was determined by fluorescent electrophoretic analysis by examining the increase in molecular weight (16%/6M urea tricine-SDS-PAGE; details of fluorescent electrophoretic analysis can be found in Schagger, H., Nat. Protocols 2006,1,16-22). The results are depicted in Figure 1, in which the upper line in the electrophoretic analysis corresponds to the glycosylated peptide. As can be seen, all of Examples 1 to 4 show the appearance of glycopeptide product bands at higher molecular weight consistent with the addition of GlcNAc to the peptide.

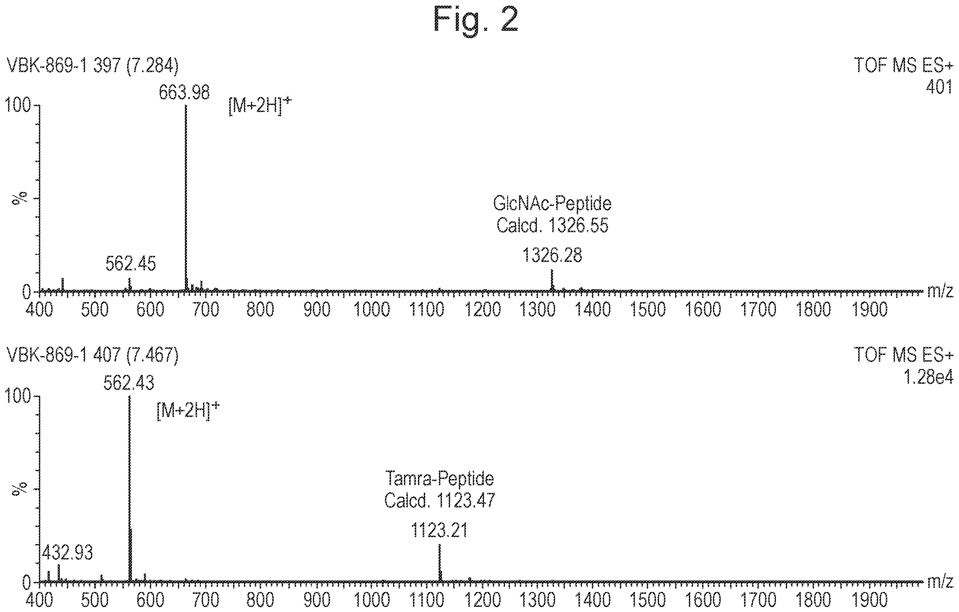

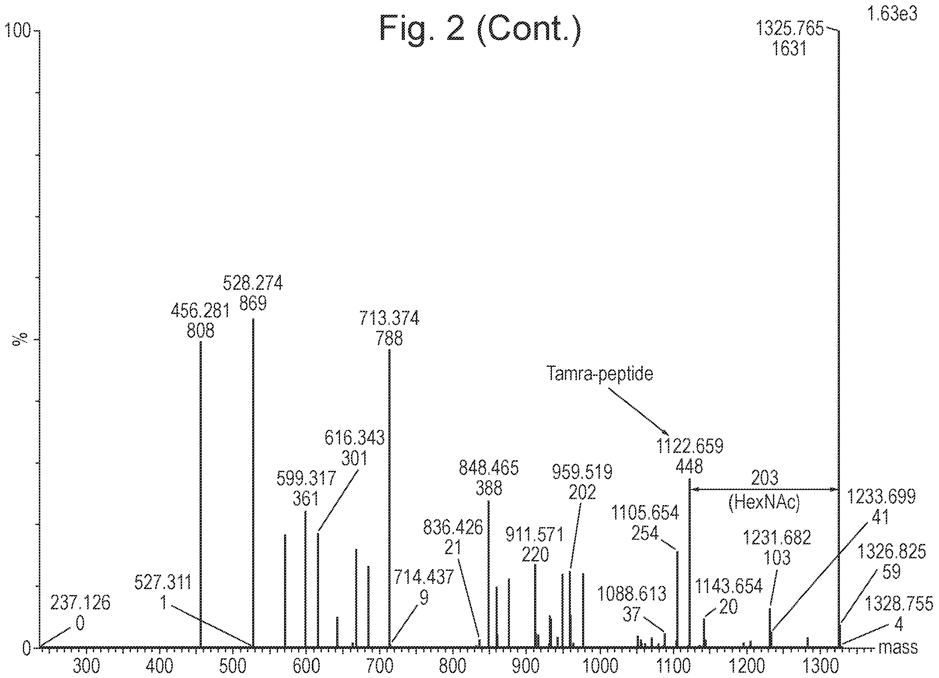

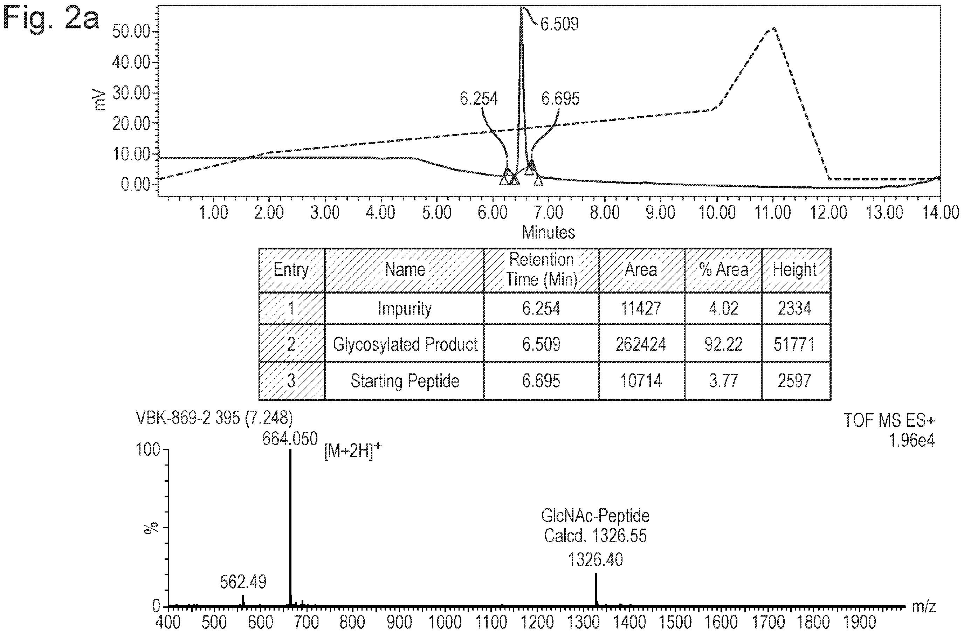

Figure 2 shows MS and MSMS analysis of the glycopeptide prepared using Example 1, again confirming the successful glycosylation of the peptide.

### Example 6a: Synthetic Optimization of Peptide Glycosylation

To a 10 µL of purified PglB (40 µM) in 1.5 mL eppendorf tube was added 34 µL of buffer (HEPES 10 mM, pH 7.5 containing 1 mM MnCl₂ and 1% Triton-X 100). 1 µL of 1mM TAMRA-Peptide and 5 µL of 1 mM Example 4 were added and the reaction mixture incubated at RT overnight. For the gel analysis, 8 µL of reaction mixture was treated with 2 µL of 4X SDS sample buffer and heated to 90°C for 5 minutes. Analytical HPLC was carried out on a Waters 2795 seperation module HPLC instrument equipped with a Jasco FP-920 Intelligent Flourescent Detector (Excitation at 542 nm, Emission at 572 nm). Phenomenex Jupiter 4µ proteo 90A (250 x 4.6 mm) column was used with a gradient of 0-20 %, 20-45%, 45-95% Acetonitrile with 0.1% formic acid at a flow rate of 1 ml/min. Product eluted with a retention time of 6.6 min and identification was performed using the Micromass LCT (TOF ESI). Conversion of the reaction was quantified by integration of the respective peak areas of product and remaining starting peptide. Results are shown in Figure 2a.

### Example 7: Kinetic Parameter Determination

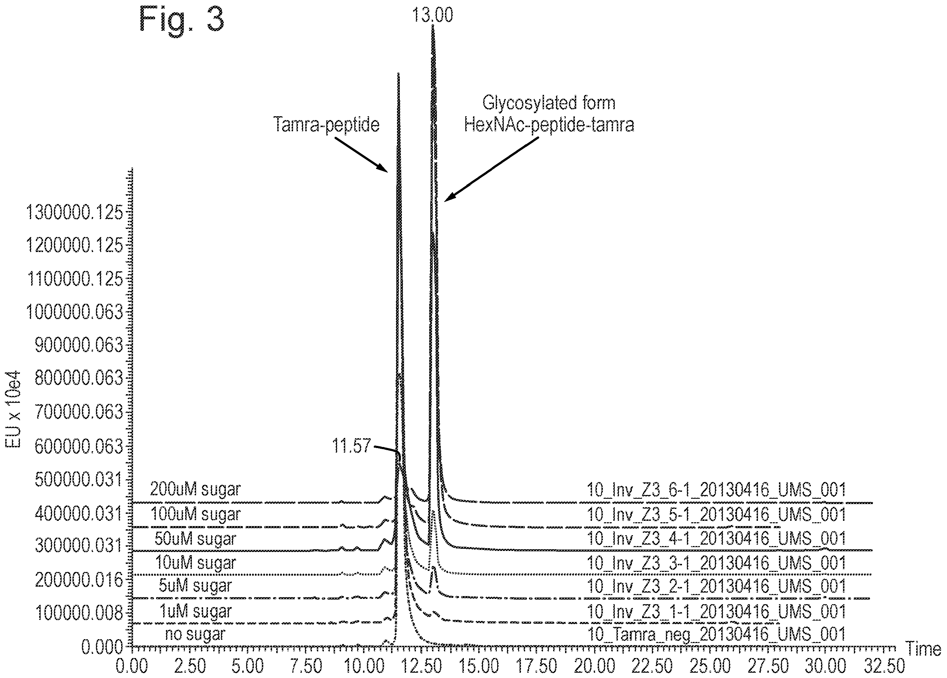

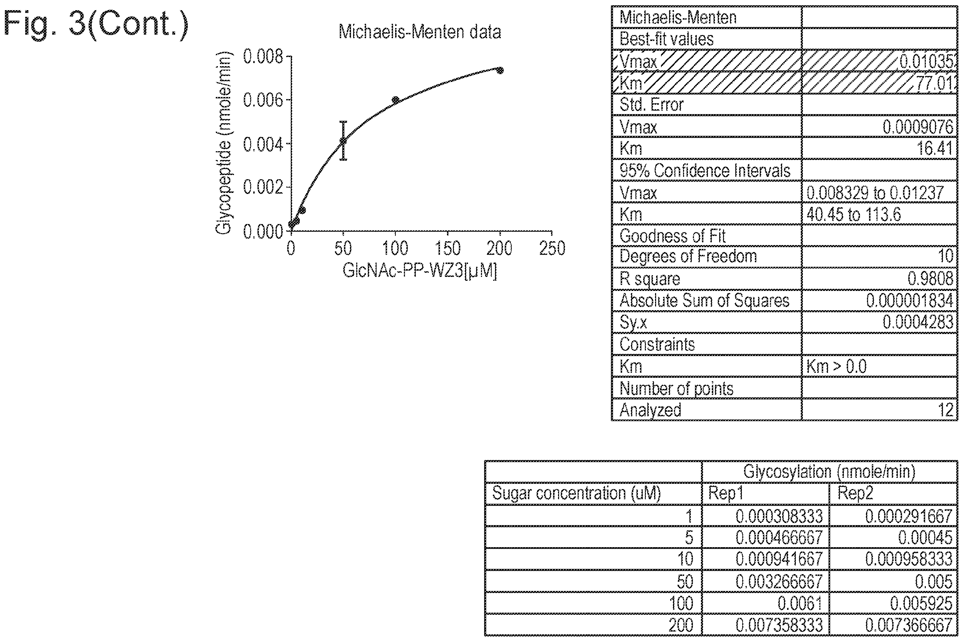

The kinetic parameters of the glycosylation reaction were further investigated by repetition (in duplicate) of the glycosylation carried out using the lipid-linked saccharide of Example 1. The glycosylation reaction was repeated as set out in Example 6 using varying concentrations of Example 1 (1, 5, 10, 50, 100 and 200 µM). Reaction mixtures were prepared as described above in Example 6 and incubated in a thermo cycler for 2 h at 30 °C. Glycosylation was terminated by flash increasing of temperature to 95 °C for 2 minutes. Peptide mixtures were cleaned up and subjected to UPLC. The separation of the glycosylated peptide from unglycosylated was achieved by normal phase using Waters Acquity UPLC column (BEH glycan, 2.1 x 150mm, 1.7-µm particles). Concentration of un- and glycosylated peptides was measured by calculating the area of corresponding peaks (Figure 3.)

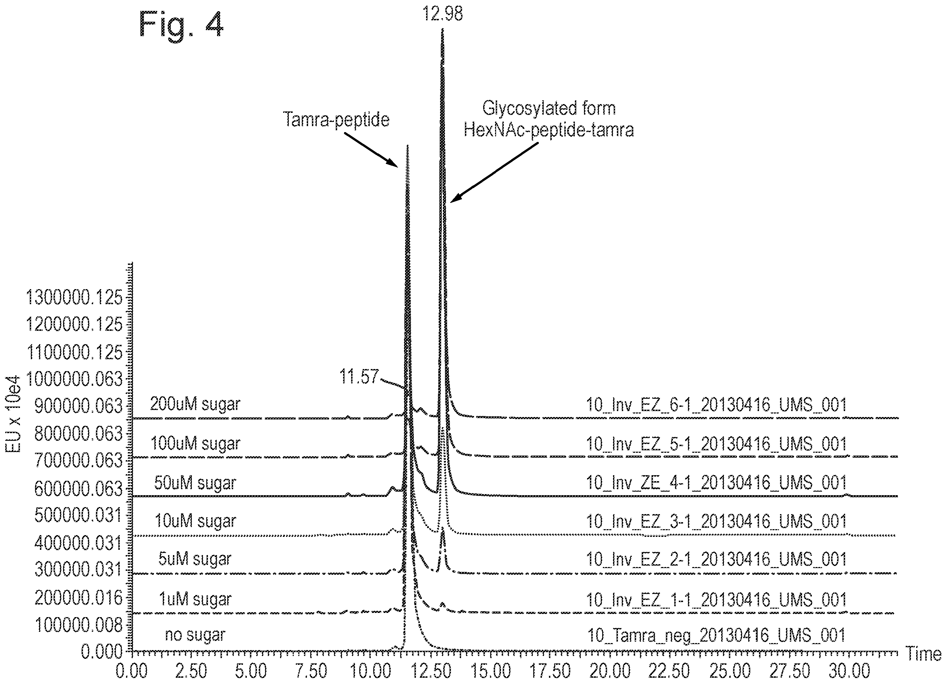

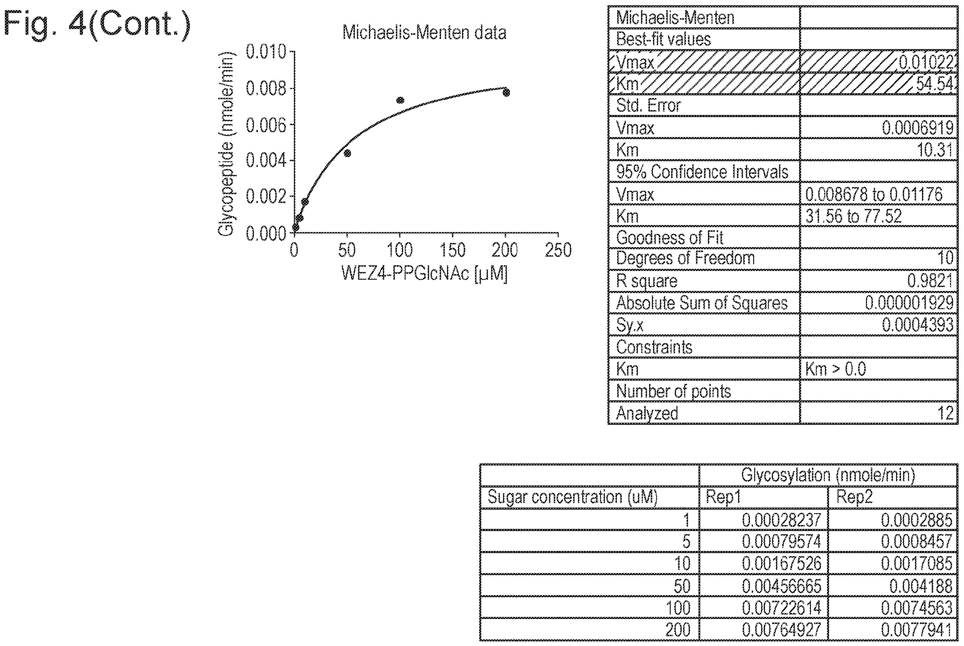

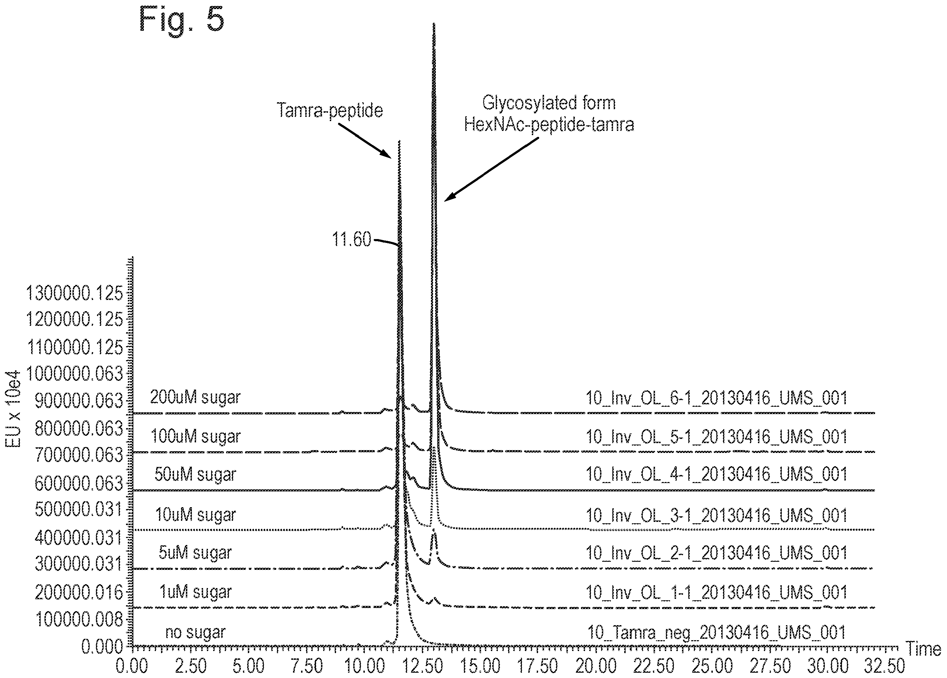

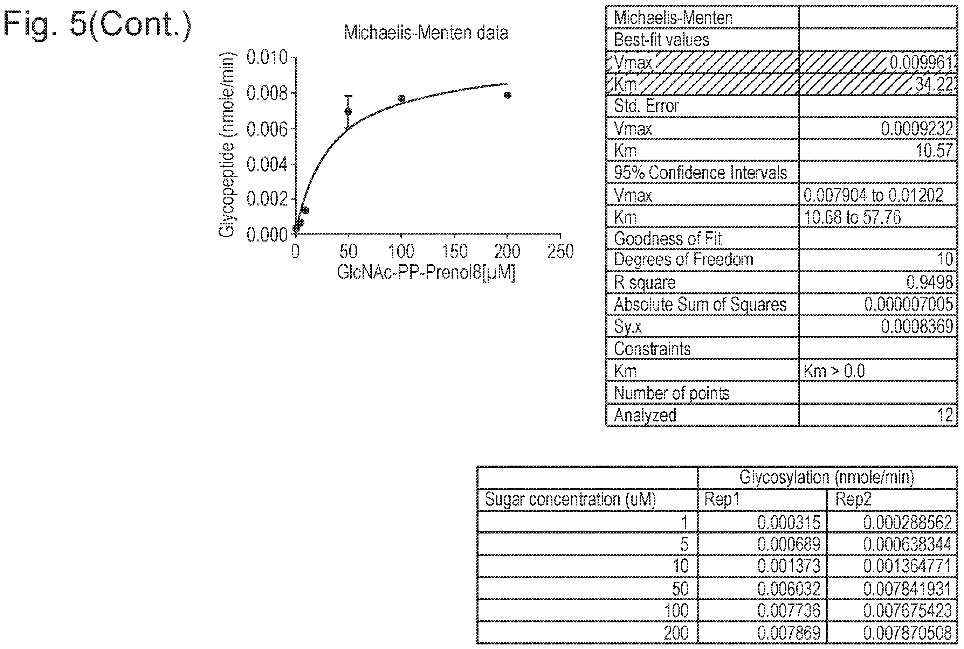

The kinetic analysis was repeated using the lipid-linked saccharide of Example 4 and comparative Example 5 and results are depicted in Figures 4 and 5 respectively.

Table 1 below sets out the kinetic parameters for PglB with GlcNAc donor glycolipid substrates of Examples 1, 4 and Comparative Example 5.

**Table 1. Kinetic Parameters for PglB with GlcNAc donor glycolipid substrates^{a}**

| Glycolipid Substrate | k_{cat} [min⁻¹] | *K*_{M} [mM] | k_{cat}/K_{M} [min⁻¹mM⁻¹] |
|---|---|---|---|
| **Ex. 1** GlcNAc-PP-WZ3 | 0.0234 ± 0.0021 | 0.077 ± 0.016 | 0.30 |
| **Ex. 4** GlcNAc-PP-WEZ4 | 0.0231 ± 0.0016 | 0.055 ± 0.01 | 0.42 |
| **C-Ex. 5** GlcNAc-PP-prenol8 | 0.0225 ± 0.0021 | 0.034 ± 0.01 | 0.66 |

| | | | |
|---|---|---|---|
| UPLC using TAMRA-fluorescence intensity; substrate concentrations: [glycolipid]= 1,5,10,50,100,200 µM, [peptide]= 20 µM; enzyme concentration [PglB] = 0.44 µM; reaction time < 2h; all conducted in duplicate. | | | |

The kinetic data suggests that in vitro reactions conducted at sufficiently high concentrations greater than K_{M} would allow transfer efficiencies equal to those found for full length lipid substrates. This is confirmed by the glycopeptide synthetic reactions being carried out at greater 90% conversion rates.

### Example 8: Endo-A Transglycosidation Glycopeptide Extension Reaction

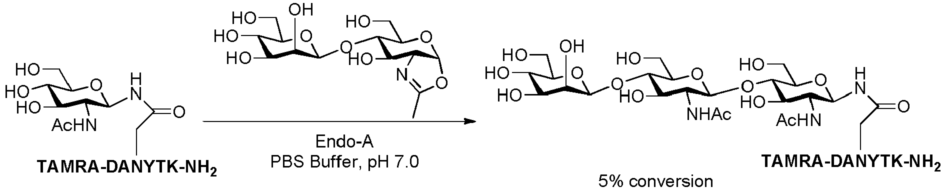

### Synthesis of ManGlcNAc₂-Peptide

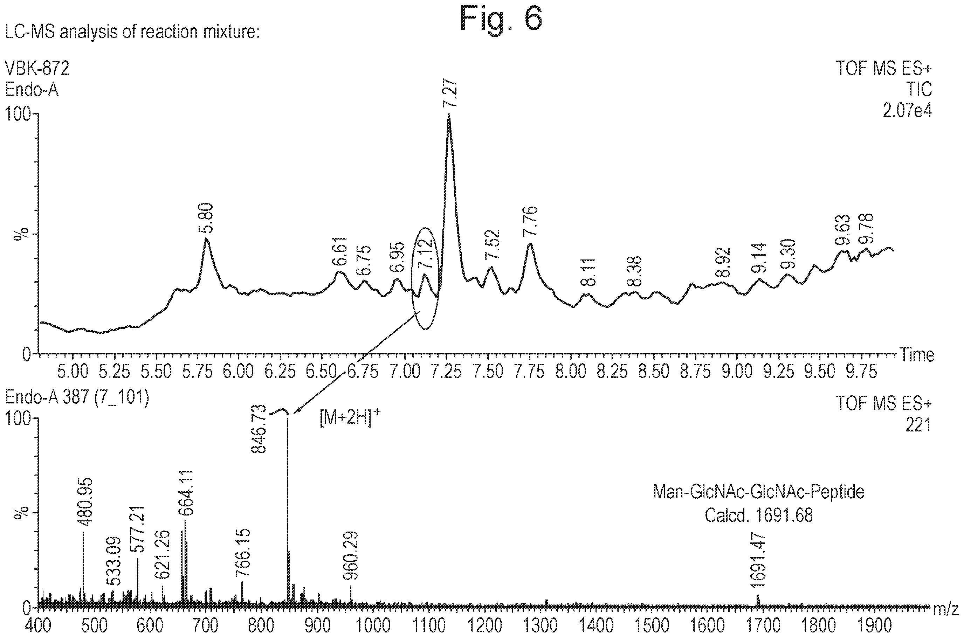

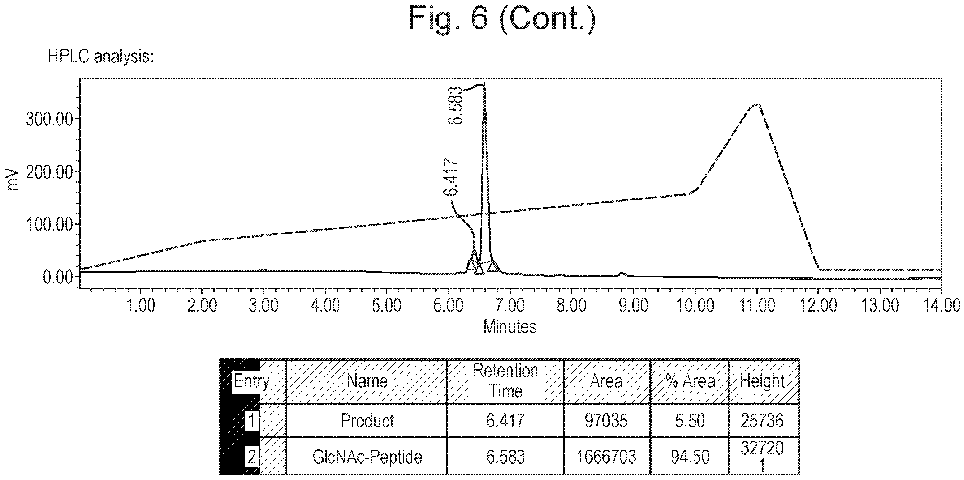

To a solution of GlcNAc-Peptide obtained in Example 6 (850 ng, 0.64 nmol) in phosphate buffer (50 mM, pH 7.0, 20 µL) in a 0.5 mL eppendorf tube was added disaccharide oxazoline (2.0 µg, 5.48 nmol, 2.7 µL of a 2 mM solution) and Endo-A (5 ug, 62.5 pmol). The reaction mixture was incubated at 30 °C for 24 hours. Product formation was analysed over a period of time by HPLC and LC-MS, as set out in Figure 6. Conversion of the reaction was quantified by integration of the respective peak areas of product and remaining GlcNAc-peptide (95%). The yield of the resulting glycosylated peptide was 5%. HRMS *m*/*z* 845.8541 (M·2[H⁺]), calculated 845.8540 for C₇₇H₁₀₅N₁₃O₃₀.

### Example-9: Endo-A Transglycosylation Glycopeptide Extension Reaction using Tetrasaccharide Oxazoline

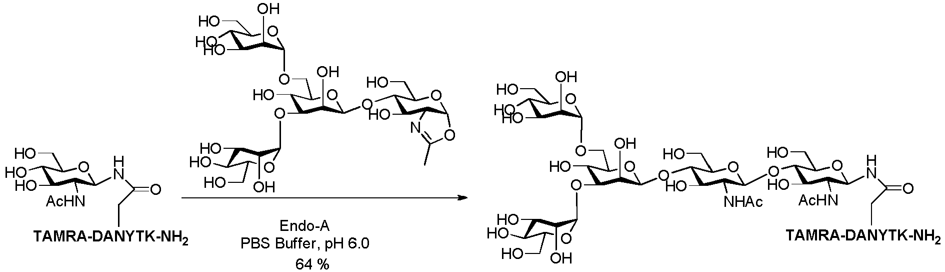

### Synthesis of Man₃GlcNAc₂-Peptide

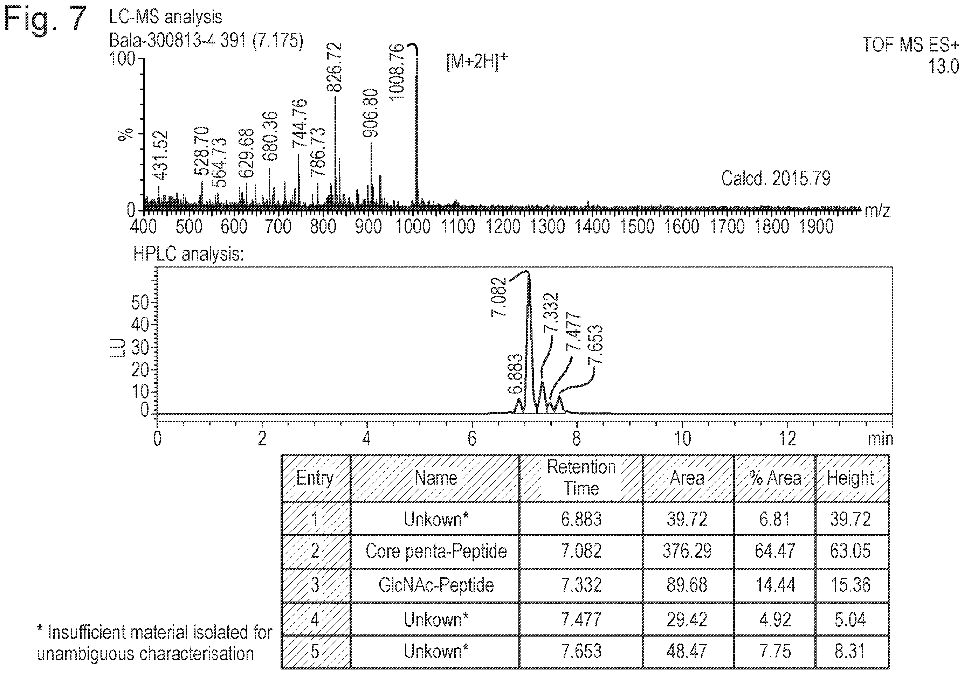

GlcNAc-Peptide obtained in Example 6 (1 µg, 0.75 nmol) in a 0.5 mL eppendorf tube containing phosphate buffer (50 mM, pH 6.0, 20 µL) was combined with tetrasaccharide oxazoline (12.6 µg, 18 nmol, 3 µL of 5 mM) and Endo-A (42 ng, 0.53 pmol) and the reaction mixture incubated at 25 °C for 2 h. Product formation was analysed by HPLC and LC-MS. Conversion of the reaction was quantified by integration of the respective peak areas of product and remaining GlcNAc-peptide. Results are depicted in Figure 7. The yield of resulting glycosylated peptide was 64%. HRMS *m*/*z* 1007.9054 (M·2[H⁺]), calculated 1007.9068 for C₈₉H₁₂₅N₁₃O₄₀.

### Example 10: Glycosidation using GalT Glycopeptide Extension Reaction

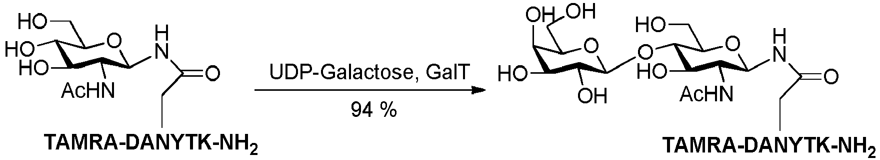

### Synthesis of GalGlcNAc-Peptide

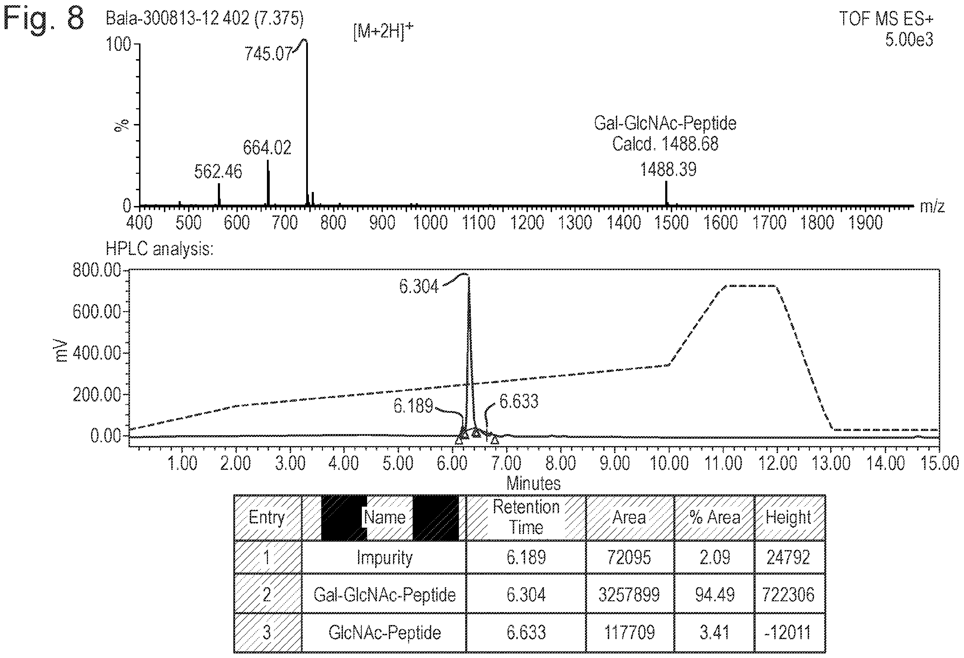

A mixture of uridine diphosphate galactose (UDP-Galactose) (6.1 µg, 10 nmol, 1µL of 10 mM), alkaline phosphatase (200 uU), galactose transferase (GalT) (600 µU) and GlcNAc-Peptide obtained in Example 6 (1 µg, 0.75 nmol) in a 100 mM sodium cacodylate, 1mg/ml BSA, 2 mM MnCl₂, pH 7.4 buffer (21 µL) was incubated at 37 °C overnight. Product formation was analysed by HPLC and LC-MS analysis and results are shown in Figure 8. Conversion of the reaction was quantified by integration of the respective peak areas of product and remaining GlcNAc peptide. The yield of resulting glycosylated peptide was 94%. HRMS (ESI⁺) *m*/*z* 744.3121 (M·2[H⁺]), calculated 744.3143 for C₆₉H₉₂N₁₂O₂₅.

### Example 11: In vitro Glycosylation of AcrA by PglB

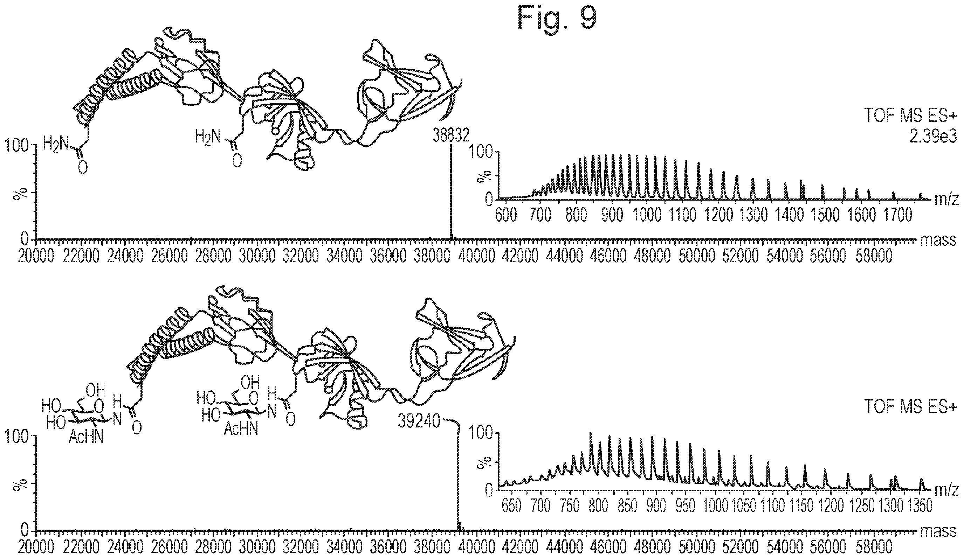

Using the process described in Example **6a** the two consensus Asn sites in the *C.jejuni* AcrA protein (overexpressed and purified from *E. coli* using pET24 (AcrA-Cys) according to the literature protocol [Kowarik, M. et. al., Science, 2006, 314, 1148-1150; Lazar, M. N. et. al., Glycobiology, 2005, 15 (4), 361-367]) were glycosylated using glycolipid substrates (Examples **2** and **4**). In a typical assay, AcrA-His₆ protein (5 µg, 129 pmol, 5 µL of 25 µM), synthetic lipid pyrophosphate sugar (Example **4**, 20.3 µg, 25.6 nmol, 12.8 µL of 2 mM) and purified PglB (0.36 µg, 10 µL of 0.44 µM) were combined in a 1.5 mL eppendorf tube with 22.2 µL of assay buffer (HEPES 10 mM, pH 7.5 containing 1 mM MnCl₂ and 1% Triton-X 100). After incubation at 25 °C for 36 hours, ES-MS analysis revealed > 95% conversion to the diglycosylated protein. ES-MS analysis of the diglycosylated protein produced using Example 4 is shown in Figure 9.

## Claims

1. A process for the production of a glycoconjugate by N-glycosylation of a protein or peptide comprising the sequence D/E-X-N-X-S/T, wherein each X is the same or different and is any natural amino acid other than proline,
wherein the process comprises reacting the protein or peptide with a polyisoprenyl pyrophosphate of formula (I), or a salt thereof, in the presence of PglB: 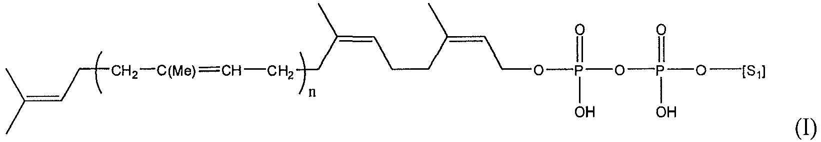
wherein n is 1, 2 or 3; and
[S1] is a monosaccharide or polysaccharide;
to produce the glycoconjugate comprising the protein or peptide having a saccharide [S1] linked to the asparagine in the sequence D/E-X-N-X-S/T.

2. A process according to claim 1, wherein the protein or peptide comprising the sequence D/E-X-N-X-S/T is an antibody.

3. A process according to any one of the preceding claims, wherein [S1] is a mono- or disaccharide.

4. A process according to any one of the preceding claims, wherein the saccharide [S1] is modified at one or more positions.

5. A process according to any one of the preceding claims, wherein [S1] is GlcNAc or GlcNAc-GlcNAc, and wherein [S1] is optionally further modified at one or more additional positions.

6. A process according to any one of the preceding claims, wherein [S1] is modified to carry one or more groups R1, wherein R1 comprises a reactive group, wherein the reactive group is an azide, alkene or alkyne group.

7. A process according to claim 6, wherein R1 comprises the reactive group and a linker moiety L.

8. A process according to claim 7, wherein L is selected from an alkylene moiety, an oxy-alkylene- moiety and a moiety of formula -NH-C(=O)- alkylene-.

9. A process according to claim 8, wherein R1 is a group of formula -(C₁-C₆ alkylene)-Y, -O-(C₁-C₆ alkylene)-Y, -NH-C(=O)-(C₁-C₆ alkylene)-Y, -NH-C(=O)-O-(C₁-C₆ alkylene)-Y, -O-C(=O)-(C₁-C₆ alkylene)-Y or -O-C(=O)-O-(C₁-C₆alkylene)-Y, wherein Y is a reactive group.

10. A process according to any one of claims 1 to 9, which process further comprises reacting the glycoconjugate with a saccharide [S2] to produce a modified glycoconjugate comprising the protein or peptide having an extended saccharide [S1-S2] linked to the asparagine in the sequence D/E-X-N-X-S/T.

11. A process according to claim 10, wherein the saccharide [S2] is a mono- or polysaccharide modified to carry a group R2, wherein R2 comprises a reactive group capable of reacting with R1 or with OH.

12. A process according to claim 11, wherein [S2] is further modified to carry one or more labelling groups.

13. A process according to claim 12, wherein the labelling group is a radioactive label selected from 18-F, 64-C and 131-1.

14. A polyisoprenyl pyrophosphate of formula (I) or a salt thereof 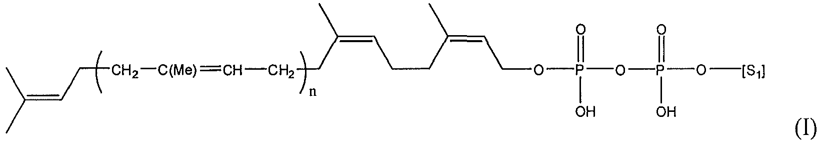 wherein n is 1, 2 or 3 and [S1] is a monosaccharide or polysaccharide wherein the saccharide unit linked to the pyrophosphate group in formula (I) is a glucose unit, and wherein the monosaccharide or polysaccharide [S1] is optionally modified at one or more positions.

15. A polyisoprenyl pyrophosphate of formula (I) according to claim 14, wherein [S1] is GlcNAc or GlcNAc-GlcNAc.

## Patentansprüche

1. Verfahren für die Herstellung eines Glykokonjugats durch N-Glykosylierung eines Proteins oder Peptids, umfassend die Sequenz D/E-X-N-X-S/T, wobei jedes X gleich oder verschieden ist und eine natürliche Aminosäure außer Prolin ist,
wobei das Verfahren ein Reagieren des Proteins oder Peptids mit einem Polyisoprenylpyrophosphat der Formel (I) oder einem Salz davon in Anwesenheit von PglB umfasst: 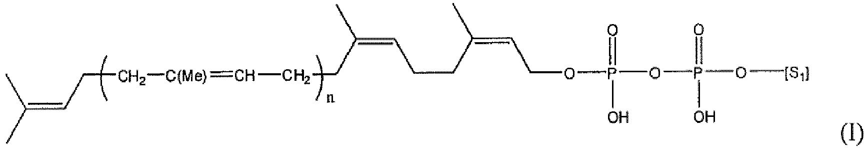
wobei n 1, 2 oder 3 ist; und
[S1] ein Monosaccharid oder Polysaccharid ist;
zum Herstellen des Glykokonjugat umfassend das Protein oder Peptid ein Saccharid [S1] aufweist, das mit dem Asparagin in der Sequenz D/E-X-N-X-S/T verbunden ist.

2. Verfahren nach Anspruch 1, wobei das Protein oder Peptid, das die Sequenz D/E-X-N-X-S/T umfasst, ein Antikörper ist.

3. Verfahren nach einem der vorherigen Ansprüche, wobei [S1] ein Mono- oder Disaccharid ist.

4. Verfahren nach einem der vorherigen Ansprüche, wobei das Saccharid [S1] an einer oder mehreren Positionen modifiziert ist.

5. Verfahren nach einem der vorherigen Ansprüche, wobei [S1] GlcNAc oder GlcNAc-GlcNAc ist, und wobei [S1] optional ferner an einer oder mehreren zusätzlichen Positionen modifiziert ist.

6. Verfahren nach einem der vorherigen Ansprüche, wobei [S1] modifiziert ist, um eine oder mehrere Gruppen R1 zu tragen, wobei R1 eine reaktive Gruppe umfasst, wobei die reaktive Gruppe eine Azid-, Alken- oder Alkingruppe ist.

7. Verfahren nach Anspruch 6, wobei R1 die reaktive Gruppe und einen Linker-Rest L umfasst.

8. Verfahren nach Anspruch 7, wobei L aus einem Alkylen-Rest, einem Oxy-alkylen-Rest und einem Rest der Formel -NH-C(=O)-Alkylen- ausgewählt ist.

9. Verfahren nach Anspruch 8, wobei R1 eine Gruppe der Formel -(C₁-C₆-Alkylen)-Y, -O-(C₁-C₆-Alkylen)-Y, -NH-C(=O)-(C₁-C₆-Alkylen)-Y, -NH-C(=O)-O-(C₁-C₆-Alkylen)-Y, -O-C(=O)-(C₁-C₆-Alkylen)-Y oder -O-C(=O)-O-(C₁-C₆-Alkylen)-Y ist, wobei Y eine reaktive Gruppe ist.

10. Verfahren nach einem der Ansprüche 1 bis 9, wobei das Verfahren ferner ein Reagieren des Glykokonjugats mit einem Saccharid [S2] umfasst, um ein modifiziertes Glykokonjugat herzustellen, das das Protein oder Peptid mit einem verlängerten Saccharid [S1-S2] umfasst, das mit dem Asparagin in der Sequenz D/E-X-N-X-S/T verbunden ist.

11. Verfahren nach Anspruch 10, wobei das Saccharid [S2] ein Mono- oder Polysaccharid ist, das modifiziert ist, um eine Gruppe R2 zu tragen, wobei R2 eine reaktive Gruppe umfasst, die in der Lage ist, mit R1 oder mit OH zu reagieren.

12. Verfahren nach Anspruch 11, wobei [S2] ferner modifiziert ist, um eine oder mehrere Markierungsgruppen zu tragen.

13. Verfahren nach Anspruch 12, wobei die Markierungsgruppe eine radioaktive Markierung ist, ausgewählt aus 18-F, 64-C und 131-1.

14. Polyisoprenylpyrophosphat der Formel (I) oder ein Salz davon 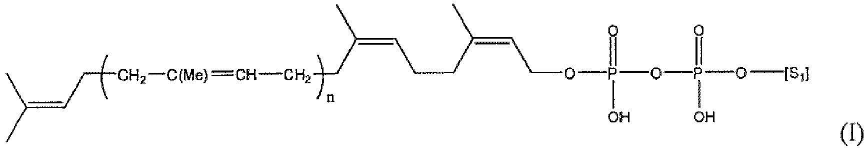 wobei n 1, 2 oder 3 ist und [S1] ein Monosaccharid oder Polysaccharid ist, wobei die mit der Pyrophosphatgruppe in Formel (I) verknüpfte Saccharideinheit eine Glukoseeinheit ist und wobei das Monosaccharid oder Polysaccharid [S1] gegebenenfalls an einer oder mehreren Positionen modifiziert ist.

15. Polyisoprenylpyrophosphat der Formel (I) nach Anspruch 14, wobei [S1] GlcNAc oder GlcNAc-GlcNAc ist.

## Revendications

1. Processus de production d'un glycoconjugué par la N-glycosylation d'une protéine ou d'un peptide comprenant la séquence D/E-X-N-X-S/T, dans lequel chaque X est similaire ou différent et est un acide aminé naturel quelconque, autre que la proline, dans lequel le processus comprend la réaction de la protéine ou du peptide avec un polyisoprényl pyrophosphate selon la formule (I), ou un sel de celui-ci, en présence de PgIB : 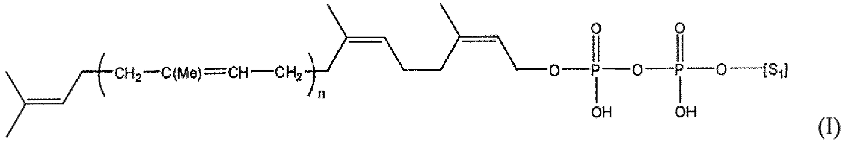
dans lequel n représente 1, 2 ou 3 ; et
[S1] est un monosaccharide ou polysaccharide ;
pour produire le glycoconjugué comprenant la protéine ou le peptide comportant un saccharide [S1] lié à l'asparagine dans la séquence D/E-X-N-X-S/T.

2. Processus selon la revendication 1, dans lequel la protéine ou le peptide comprenant la séquence D/E-X-N-X-S/T est un anticorps.

3. Processus selon l'une quelconque des revendications précédentes, dans lequel [S1] est un mono ou un disaccharide.

4. Processus selon l'une quelconque des revendications précédentes, dans lequel le saccharide [S1] est modifié au niveau d'une ou plusieurs positions.

5. Processus selon l'une quelconque des revendications précédentes, dans lequel [S1] est GlcNAc ou GlcNAc-GlcNAc, et dans lequel [S1] est en outre éventuellement modifié au niveau d'une ou plusieurs positions supplémentaires.

6. Processus selon l'une quelconque des revendications précédentes, dans lequel [S1] est modifié pour porter un ou plusieurs groupes R1, dans lequel R1 comprend un groupe réactif, dans lequel le groupe réactif est un groupe azide, alcène ou alcyne.

7. Processus selon la revendication 6, dans lequel R1 comprend le groupe réactif et une fraction de liaison L.

8. Processus selon la revendication 7, dans lequel L est choisi parmi une fraction alkylène, une fraction oxy-alkylène et une fraction de formule -NH-C(=O)-alkylène-.

9. Processus selon la revendication 8, dans lequel R1 est un groupe de formule -(alkylène en C₁-C₆)-Y, -O-(alkylène en C₁-C₆)-Y, -NH-C(=O)-(alkylène en C₁-C₆)-Y, -NH-C(=O)-O-(alkylène en C₁-C₆)-Y, -O-C(=O)-(alkylène en C₁-C₆)-Y ou -O-C(=O)-O-(alkylène en C₁-C₆)-Y, dans lequel Y est un groupe réactif.

10. Processus selon l'une quelconque des revendications 1 à 9, dont le processus comprend en outre la réaction du glycoconjugué avec un saccharide [S2] pour produire un glycoconjugué modifié comprenant la protéine ou le peptide possédant un saccharide étendu [S1-S2] lié à l'asparagine dans la séquence D/E-X-N-X-S/T.

11. Processus selon la revendication 10, dans lequel le saccharide [S2] est un mono ou un polysaccharide modifié pour porter un groupe R2, dans lequel R2 comprend un groupe réactif en mesure de réagir avec R1 ou OH.

12. Processus selon la revendication 11, dans lequel [S2] est en outre modifié pour porter un ou plusieurs groupes de marquage.

13. Processus selon la revendication 12, dans lequel le groupe de marquage est un traceur radioactif choisi parmi 18-F, 64-C et 131-I.

14. Polyisoprényl pyrophosphate de formule (I) ou sel de celui-ci 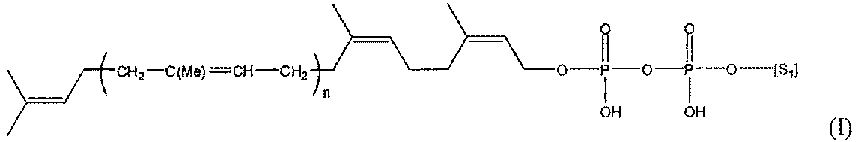 dans lequel n représente 1, 2 ou 3 et [S1] est un monosaccharide ou un polysaccharide dans lequel l'unité de saccharide liée au groupe de pyrophosphate dans la formule (I) est une unité de glucose, et dans lequel le monosaccharide ou le polysaccharide [S1] est éventuellement modifié au niveau d'une ou plusieurs positions.

15. Polyisoprényl pyrophosphate de formule (I) selon la revendication 14, dans lequel [S1] est GlcNAc ou GlcNAc-GlcNAc.
